(19) Europäisches Patentamt
European Patent Office
Office européen des brevets

(11) EP 4 461 763 A1

(12) EUROPEAN PATENT APPLICATION
published in accordance with Art. 153(4) EPC

(43) Date of publication:
**13.11.2024 Bulletin 2024/46**

(21) Application number: **22918840.4**

(22) Date of filing: **22.12.2022**

(51) International Patent Classification (IPC):
***C08G 77/46*** (2006.01) ***A61K 8/04*** (2006.01)
***A61Q 1/00*** (2006.01) ***A61Q 5/00*** (2006.01)
***A61Q 17/04*** (2006.01) ***A61Q 19/00*** (2006.01)
***C08G 77/38*** (2006.01)

(52) Cooperative Patent Classification (CPC):
**A61K 8/04; A61Q 1/00; A61Q 5/00; A61Q 17/04; A61Q 19/00; C08G 77/38; C08G 77/46**

(86) International application number:
**PCT/JP2022/047303**

(87) International publication number:
**WO 2023/132256 (13.07.2023 Gazette 2023/28)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **05.01.2022 JP 2022000545**

(71) Applicant: **SHIN-ETSU CHEMICAL CO., LTD.**
**Tokyo 1000005 (JP)**

(72) Inventor: **IMAI Taro**
**Annaka-shi, Gunma 379-0224 (JP)**

(74) Representative: **Mewburn Ellis LLP**
**Aurora Building**
**Counterslip**
**Bristol BS1 6BX (GB)**

(54) **(POLY)GLYCERIN GROUP- AND POLYOXYALKYLENE GROUP-CONTAINING ORGANOPOLYSILOXANE, TREATED HYDROPHOBIC POWDER, DISPERSION, AND COSMETIC**

(57) Provided are: an organopolysiloxane that, when used to treat a hydrophobic powder, imparts excellent dispersibility in water to the treated hydrophobic powder; a treated hydrophobic powder; a dispersion; and a cosmetic.

The organopolysiloxane contains a (poly)glycerin group and a polyoxyalkylene group.



Processed by Luminess, 75001 PARIS (FR)

## Description

### TECHNICAL FIELD

This invention relates to an organopolysiloxane containing (poly)glycerin and polyoxyalkylene groups, a treated hydrophobic powder treated with the organopolysiloxane, and a dispersion and a cosmetic composition containing the powder.

### BACKGROUND ART

Since oil-in-water (O/W) type emulsified cosmetics whose continuous phase is water give a fresh and/or light feeling on use, they win consumers' favor as the current tendency. In general, a variety of powders are blended in cosmetics for the purposes of improving feeling on use and achieving a UV-scattering effect. However, since the powder blended in the water phase of O/W type emulsified cosmetics is hydrophilic, films of the cosmetics are less resistant to sweat and water, leaving the problem of water resistance.

As the countermeasure, an attempt was made to blend oil-treated hydrophobic powder in the water phase. There are some advantages that not only the cosmetic film is improved in water resistance, but the adhesion to the skin is also improved as compared with the blending of hydrophobic powder in the oil phase because water in the continuous phase is prone to volatilize. For example, Patent Document 1: JP-A 2018-024881 discloses a method of blending a hydrophobic powder in a water phase using a polyoxyalkylene-modified silicone as a powder dispersant. However, it is difficult to disperse the hydrophobic powder in water. The powder in the water phase agglomerates together with the lapse of time, giving rise to problems in usability and feeling on use. Patent Document 2: WO 2016/056589 and Patent Document 3: JP-A 2017-137252 disclose methods of establishing dispersion stability by blending a water-swelling substance or water-soluble polymer. However, when the water-swelling substance or water-soluble polymer is blended in a sufficient amount to establish stability, the feeling on the skin becomes more sticky or oily, that is, the feeling on use is aggravated. In addition, when the hydrophobic powder is heavily loaded, the dispersion has so high a viscosity as to give a poor feeling on use.

Further, Patent Document 4: JP-A 2018-115211 discloses to use a carboxylic acid-modified silicone as a dispersant. This technique, however, fails to control a viscosity buildup with the lapse of time. Since the dispersion must be kept at a neutral or alkaline pH, the formulation of a cosmetic composition is restricted.

### PRIOR ART DOCUMENTS

### PATENT DOCUMENTS

Patent Document 1: JP-A 2018-024881
Patent Document 2: WO 2016/056589
Patent Document 3: JP-A 2017-137252
Patent Document 4: JP-A 2018-115211

### SUMMARY OF INVENTION

### TECHNICAL PROBLEM

An object of the invention, which has been made under the above-mentioned circumstances, is to provide an organopolysiloxane with which a hydrophobic powder is treated so that the treated hydrophobic powder may be effectively dispersed in water, a treated hydrophobic powder, and a dispersion and a cosmetic composition.

Making extensive investigations to attain the above object, the inventor has found that a treated hydrophobic powder is obtained by treating a hydrophobic powder with an organopolysiloxane containing specific (poly)glycerin and polyoxyalkylene groups, i.e., a (poly)glycerin group which is fully adsorptive to powder and a polyoxyalkylene group which is fully compatible with water phase, the treated hydrophobic powder is effectively dispersed in water, and a dispersion having the treated hydrophobic powder blended therein has a low viscosity and excellent age stability. Since the hydrophobic powder is effectively dispersed, a dispersion of the treated hydrophobic powder and a cosmetic composition comprising the dispersion give a satisfactory fresh feeling on use, and form a film (cosmetic film) on the skin which is fully uniform due to effective dispersion of the hydrophobic powder, and provides excellent water resistance because the hydrophobic powder is blended. The invention is predicated on this finding.

Accordingly, the invention provides an organopolysiloxane containing (poly)glycerin and polyoxyalkylene

groups, a treated hydrophobic powder, a dispersion, and a cosmetic composition as defined below.

1. An organopolysiloxane containing (poly)glycerin and polyoxyalkylene groups, represented by the compositional formula (1):
[Chem. 1]

$$(R^1{}_2SiO_{2/2})_a(R^1R^2SiO_{2/2})_b(R^1R^3SiO_{2/2})_c(R^1R^4SiO_{2/2})_d(R^1R^5SiO_{2/2})_e(R^1SiO_{3/2})_f(SiO_{4/2})_g(R^1{}_3SiO_{1/2})_{2+f+2g} \quad (1)$$

wherein $R^1$ is independently a monovalent hydrocarbon group selected from $C_1$-$C_5$ alkyl groups, $C_6$-$C_{15}$ aryl groups, and $C_7$-$C_{15}$ aralkyl groups,

$R^2$ is a $C_6$-$C_{20}$ alkyl group,
$R^3$ is a (poly)glycerin group having the general formula (2):

[Chem. 2]

$$-C_hH_{2h}-O\left(CH_2\overset{\overset{\displaystyle OH}{|}}{C}HCH_2O\right)_i-H \quad (2)$$

wherein h is an integer satisfying $0 \leq h \leq 20$, and i satisfies $1 \leq i \leq 10$,
$R^4$ is a polyoxyalkylene group having the general formula (3):
[Chem. 3]

$$-C_jH_{2j}-O-(C_2H_4O)_{k1}-(C_3H_6O)_{k2}-R^6 \quad (3)$$

wherein $R^6$ is hydrogen or a $C_1$-$C_{30}$ hydrocarbon group, j is an integer satisfying $0 \leq j \leq 20$, k1 and k2 satisfy $2 \leq k1 \leq 100$ and $0 \leq k2 \leq 100$,
$R^5$ is a group having the general formula (4), (5), (6) or (7):
[Chem. 4]

$$-(CH_2)_2-C_mH_{2m}-(SiOR^1{}_2)_n-SiR^1{}_3 \quad (4)$$

$$-(CH_2)_2-C_mH_{2m}-SiR^1{}_{n1}-(OSiR^1{}_3)_{3-n1} \quad (5)$$

$$-(CH_2)_2-C_mH_{2m}-SiR^1{}_{n1}-(OSiR^1{}_{n2}(OSiR^1{}_3)_{3-n2})_{3-n1} \quad (6)$$

$$-(CH_2)_2-C_mH_{2m}-SiR^1{}_{n1}-(OSiR^1{}_{n2}(OSiR^1{}_{n3}(OSiR^1{}_3)_{3-n3})_{3-n2})_{3-n1} \quad (7)$$

wherein $R^1$ is as defined above, m and n each are an integer satisfying $0 \leq m \leq 5$ and $0 \leq n \leq 100$, n1 to n3 each are an integer satisfying $0 \leq n1 \leq 2$, $0 \leq n2 \leq 2$, and $0 \leq n3 \leq 2$, a, b, c, d, e, f, and g each satisfy

$$0 \leq a \leq 50,$$

$$0 \leq b \leq 20,$$

$$1 \leq c \leq 10,$$

$$1 \leq d \leq 10,$$

$$0 \le e \le 10,$$

$$f \ge 0, \text{ and } g \ge 0.$$

2. The organopolysiloxane of 1 wherein in compositional formula (1), the ratio of c/d is $0.1 \le (c/d) \le 1.5$.
3. A treated hydrophobic powder comprising (B) a hydrophobic powder which is treated with (A) the organopolysiloxane of 1 or 2.
4. A dispersion comprising the treated hydrophobic powder of 3.
5. A cosmetic composition comprising the treated hydrophobic powder of 3 or the dispersion of 4.
6. The cosmetic composition of 5, further comprising (D) a polyhydric alcohol.
7. The cosmetic composition of 5, further comprising (E) an oil.

## ADVANTAGEOUS EFFECTS OF INVENTION

The treated hydrophobic powder is obtained by treating a hydrophobic powder with an organopolysiloxane containing (poly)glycerin and polyoxyalkylene groups. The treated hydrophobic powder can be effectively dispersed and blended in water. The dispersion having the treated hydrophobic powder blended therein has a low viscosity and excellent age stability. The dispersion of the treated hydrophobic powder and a cosmetic composition having the dispersion blended therein give a pleasant fresh feeling on use and can form a film (or cosmetic film) having improved water resistance on the skin.

## DESCRIPTION OF EMBODIMENTS

Now the invention is described in detail.

[Component (A)]

Component (A) is an organopolysiloxane containing (poly)glycerin and polyoxyalkylene groups, represented by the compositional formula (1):
[Chem. 5]

$$(R^1{}_2SiO_{2/2})_a(R^1R^2SiO_{2/2})_b(R^1R^3SiO_{2/2})_c(R^1R^4SiO_{2/2})_d(R^1R^5SiO_{2/2})_e(R^1SiO_{3/2})_f(SiO_{4/2})_g(R^1{}_3SiO_{1/2})_{2+f+2g} \quad (1)$$

wherein $R^1$ is independently a monovalent hydrocarbon group selected from $C_1$-$C_5$ alkyl groups, $C_6$-$C_{15}$ aryl groups, and $C_7$-$C_{15}$ aralkyl groups,

$R^2$ is a $C_6$-$C_{20}$ alkyl group,
$R^3$ is a (poly)glycerin group having the general formula (2):

[Chem. 6]

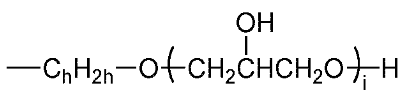

(2)

wherein h is an integer satisfying $0 \le h \le 20$, and i satisfies $1 \le i \le 10$,
$R^4$ is a polyoxyalkylene group having the general formula (3):
[Chem. 7]

$$-C_jH_{2j}-O-(C_2H_4O)_{k1}-(C_3H_6O)_{k2}-R^6 \quad (3)$$

wherein $R^6$ is hydrogen or a $C_1$-$C_{30}$ hydrocarbon group, j is an integer satisfying $0 \le j \le 20$, k1 and k2 satisfy $2 \le k1 \le 100$ and $0 \le k2 \le 100$,
$R^5$ is a group having the general formula (4), (5), (6) or (7):

[Chem. 8]

$-(CH_2)_2-C_mH_{2m}-(SiOR^1{}_2)_n-SiR^1{}_3$ (4)

$-(CH_2)_2-C_mH_{2m}-SiR^1{}_{n1}-(OSiR^1{}_3)_{3-n1}$ (5)

$-(CH_2)_2-C_mH_{2m}-SiR^1{}_{n1}-(OSiR^1{}_{n2}(OSiR^1{}_3)_{3-n2})_{3-n1}$ (6)

$-(CH_2)_2-C_mH_{2m}-SiR^1{}_{n1}-(OSiR^1{}_{n2}(OSiR^1{}_{n3}(OSiR^1{}_3)_{3-n3})_{3-n2})_{3-n1}$ (7)

wherein $R^1$ is as defined above, m and n each are an integer satisfying $0 \le m \le 5$ and $0 \le n \le 100$, n1 to n3 each are an integer satisfying $0 \le n1 \le 2$, $0 \le n2 \le 2$, and $0 \le n3 \le 2$, a, b, c, d, e, f, and g each satisfy

$$0 \le a \le 50,$$

$$0 \le b \le 20,$$

$$1 \le c \le 10,$$

$$1 \le d \le 10,$$

$$0 \le e \le 10,$$

$$f \ge 0, \text{ and } g \ge 0.$$

The organopolysiloxane may be used alone or in admixture of two or more.

$R^1$ is independently a monovalent hydrocarbon group selected from $C_1$-$C_5$ alkyl groups, $C_6$-$C_{15}$ aryl groups, and $C_7$-$C_{15}$ aralkyl groups. Exemplary groups include alkyl groups such as methyl, ethyl, propyl, butyl and pentyl, cycloalkyl groups such as cyclopentyl, aryl groups such as phenyl and tolyl, and aralkyl groups such as benzyl and phenethyl. It is acceptable that different groups are contained per molecule. $R^1$ is preferably methyl.

$R^2$ is a $C_6$-$C_{20}$ alkyl group which may be straight or branched. It is acceptable that different groups are contained per molecule. Exemplary groups include hexyl, heptyl, octyl, nonyl, decyl, dodecyl, tetradecyl, and hexadecyl. Although a suitable group may be selected for $R^2$ depending on the type of a surface treating agent for hydrophobic powder to be described later, dodecyl and hexadecyl are preferred in view of compatibility with the hydrophobic powder surface, with dodecyl, i.e., an alkyl group of 12 carbon atoms being more preferred. Where the organopolysiloxane contains $R^2$, the content of $R^2$ is preferably 5 to 40% by weight, more preferably 10 to 30% by weight of the overall weight of the organopolysiloxane.

$R^3$ is a (poly)glycerin group having the general formula (2):

[Chem. 9]

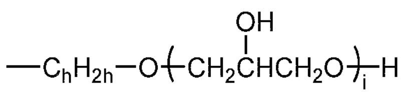 (2)

wherein h is an integer satisfying $0 \le h \le 20$, and i satisfies $1 \le i \le 10$. It is acceptable that different groups are contained per molecule. While h is an integer satisfying $0 \le h \le 20$, examples of the relevant structure include $-(CH_2)_2-$, $-(CH_2)_3-$, $-CH_2CH(CH_3)CH_2-$, $-(CH_2)_4-$, $-(CH_2)_5-$, $-(CH_2)_6-$, $-(CH_2)_7-$, $-(CH_2)_8-$, $-(CH_2)_2-CH(CH_2CH_2CH_3)-$, and $-CH_2-CH(CH_2CH_3)-$. The subscript i satisfies $1 \le i \le 10$, and is preferably 2 to 5, more preferably 2 to 3. If i exceeds 10, the organopolysiloxane itself has so high a viscosity as to aggravate feeling on use or usability. When the

organopolysiloxane is used as a dispersant for dispersing hydrophobic powder in a water phase, the content of $R^3$ is preferably 5 to 30% by weight, more preferably 5 to 20% by weight of the overall weight of the organopolysiloxane. As used herein, the term "(poly)glycerin group" encompasses monoglycerin and polyglycerin groups as well as structural isomers thereof. Examples of $R^3$ having formula (2) wherein h=3 and i=1 to 3 include groups derived from monoglycerin having the general formula (8), diglycerins having the general formulae (9) and (10), and triglycerins having the general formulae (11) to (14).

[Chem. 10]

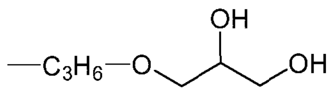

(8)

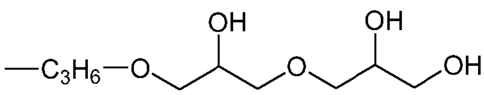

(9)

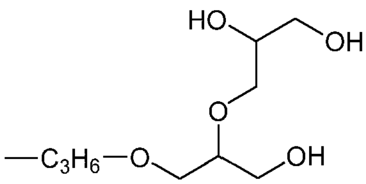

(10)

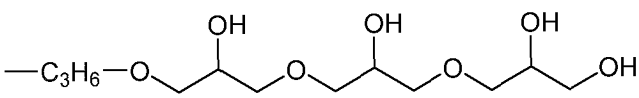

(11)

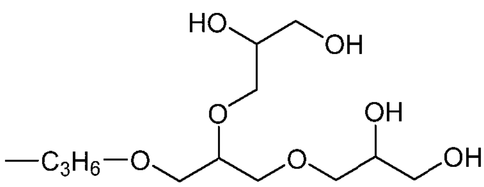

(12)

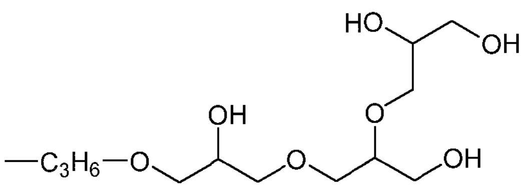

(13)

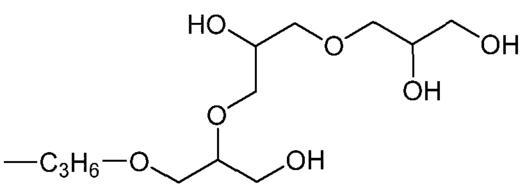

(14)

$R^4$ is a polyoxyalkylene group having the general formula (3):
[Chem. 11]

$-C_jH_{2j}-O-(C_2H_4O)_{k1}-(C_3H_6O)_{k2}-R^6$ (3)

wherein $R^6$ is hydrogen or a $C_1$-$C_{30}$ hydrocarbon group, j is an integer satisfying $0 \leq j \leq 20$, k1 and k2 satisfy $2 \leq k1 \leq 100$ and $0 \leq k2 \leq 100$. It is acceptable that different groups are contained per molecule.

$R^6$ is hydrogen or a $C_1$-$C_{30}$ hydrocarbon group, examples of which include $-(CH_2)_2-$, $-(CH_2)_3-$, $-CH_2CH(CH_3)CH_2-$, $-(CH_2)_4-$, $-(CH_2)_5-$, $-(CH_2)_6-$, $-(CH_2)_7-$, $-(CH_2)_8-$, $-(CH_2)_2-CH(CH_2CH_2CH_3)-$, and $-CH_2-CH(CH_2CH_3)-$. The subscript j is an integer satisfying $0 \leq j \leq 20$, preferably $1 \leq j \leq 5$; k1 satisfies $2 \leq k1 \leq 100$, preferably $2 \leq k1 \leq 50$, more preferably $2 \leq k1 \leq 15$; and k2 satisfies $0 \leq k2 \leq 100$, preferably $0 \leq k2 \leq 50$, more preferably $0 \leq k2 \leq 5$. When the organopolysiloxane is used as a dispersant for dispersing hydrophobic powder in a water phase, the content of $R^4$ is preferably 20 to 70% by weight, more preferably 40 to 60% by weight of the overall weight of the organopolysiloxane.

$R^5$ is a group having the general formula (4), (5), (6) or (7):

[Chem. 12]

$-(CH_2)_2-C_mH_{2m}-(SiOR^1_2)_n-SiR^1_3$ (4)

$-(CH_2)_2-C_mH_{2m}-SiR^1_{n1}-(OSiR^1_3)_{3-n1}$ (5)

$-(CH_2)_2-C_mH_{2m}-SiR^1_{n1}-(OSiR^1_{n2}(OSiR^1_3)_{3-n2})_{3-n1}$ (6)

$-(CH_2)_2-C_mH_{2m}-SiR^1_{n1}-(OSiR^1_{n2}(OSiR^1_{n3}(OSiR^1_3)_{3-n3})_{3-n2})_{3-n1}$ (7)

wherein $R^1$ is as defined above, m and n each are an integer satisfying $0 \leq m \leq 5$ and $0 \leq n \leq 100$, n1 to n3 each are an integer satisfying $0 \leq n1 \leq 2$, $0 \leq n2 \leq 2$, and $0 \leq n3 \leq 2$. It is acceptable that different groups are contained per molecule.

The subscript m is an integer satisfying $0 \leq m \leq 5$. Particularly in case of synthesis from reaction of vinylsiloxy group with hydrosilyl group, m is 0. The subscript n is an integer satisfying $0 \leq n \leq 100$, preferably from 1 to 30. The subscripts n1 to n3 are integers satisfying $0 \leq n1 \leq 2$, $0 \leq n2 \leq 2$, and $0 \leq n3 \leq 2$.

When component (A) is used as a dispersant, the ratio (c/d) in formula (1) is preferably $0.1 \leq (c/d) \leq 1.5$, more preferably $0.2 \leq (c/d) \leq 0.8$. When the ratio (c/d) is 0.1 or more, the organopolysiloxane is more adsorptive to the hydrophobic powder surface. When the ratio (c/d) is 1.5 or less, the organopolysiloxane is restrained from viscosity buildup, leading to improvements in usability and dispersibility.

The organopolysiloxane (A) is synthesized by hydrosilylation reaction of hydrogenpolysiloxane having a hydrosilyl group serving as a reactive site with a compound having an alkenyl group (or carbon-carbon unsaturated bond) at the end as shown below. For the hydrosilylation reaction, any well-known methods may be employed. The organopolysiloxane (A) may be prepared while its construction is adjusted by selecting the type and amount of reactants.

[Chem. 13] $CH_2=CH-R^1$ $CH_2=CH-R^2$ $CH_2=CH-R^3$ $CH_2=CH-C_mH_{2m}-(SiOR^1_2)_n-SiR^1_3$ $CH_2=CH-C_mH_2-SiR^1_{n1}-(OSiR^1_3)_{3-n1}$ $CH_2=CH-C_mH_2-SiR^1_{n1}-(OSiR^1_{n2}(OSiR^1_3)_{3-n2})_{3-n1}$ $CH_2=CH-C_mH_2-SiR^1_{n1}-(OSiR^1_{n2}(OSiR^1_{n3}(OSiR^1_3)_{3-n3})_{3-n2})_{3-n1}$

Herein, $R^1$ to $R^4$, m and n are as defined above.

[Treated hydrophobic powder treated with organopolysiloxane (A)]

Another embodiment of the invention is a treated hydrophobic powder comprising (B) a hydrophobic powder which is treated with (A) the organopolysiloxane. Component (A) is used as a dispersant for component (B). Suitable treatment methods include a method of mixing the organopolysiloxane (A) with the hydrophobic powder (B) and a method of preparing a dispersion as described below. The mixing method is not particularly limited and the mixing temperature, time, means and the like may be selected as appropriate. In the mixing step, (D) a polyhydric alcohol such as 1,3-butylene glycol may be used as well as another surfactant.

The amount of organopolysiloxane (A) used for the treatment of hydrophobic powder (B) is preferably such that the weight ratio (A)/(B) may range from 0.01/1 to 1/1, more preferably from 0.02/1 to 0.5/1.

[Component (B)]

Component (B) is a hydrophobic powder. The hydrophobic powder used herein may have any shape, for example, spherical, spindle, needle or plate shape, any particle size, for example, fumed, microparticulate, or pigment grade, and any particle structure, for example, porous or non-porous as long as it is used in conventional cosmetics. The hydrophobic powder may be used alone or in admixture of two or more.

The hydrophobic powder (B) comprises a source powder serving as base particles which is treated on the surface

with a hydrophobic treatment agent. It is noted that when the source powder prior to treatment is hydrophobic on the surface, it may be used without hydrophobic treatment. As used herein, the term "hydrophobic" modifying the hydrophobic powder means that the substance is not dispersible in water.

Examples of the source powder serving as base particles of the hydrophobic powder include microparticulate metal oxide powders, coloring pigments, inorganic powders, metal powders, organic powders, and inorganic/organic composite powders.

The microparticulate metal oxide is selected from microparticulate titanium oxide (labeling name, INCI name: Titanium Dioxide), iron-containing titanium oxide, zinc oxide (labeling name, INCI name: Zinc Oxide), cerium oxide (labeling name, INCI name: Cerium Oxide), and composites thereof. Any of these metal oxides may be a composite powder with another powder.

The coloring pigment is not particularly limited as long as it is commonly used in cosmetics for the purpose of coloration. Any of the following pigments may be used, for example, red iron oxide (labeling name, INCI name: Iron Oxides), yellow iron oxide (labeling name, INCI name: Iron Oxides), white titanium oxide (labeling name, INCI name: Titanium Dioxide), black iron oxide (labeling name, INCI name: Iron Oxides), ultramarine (labeling name, INCI name: Ultramarines), Prussian blue (labeling name, INCI name: Ferric Ferrocyanide, Ferric Ammonium Ferrocyanide), manganese violet (labeling name, INCI name: Manganese Violet), cobalt titanate (labeling name, INCI name: Cobalt Titanium Oxide), chromium hydroxide (labeling name, INCI name: Chromium Hydroxide Green), chromium oxide (labeling name, INCI name: Chromium Oxide Greens), Al/Co oxide (labeling name, INCI name: Cobalt Aluminum Oxide), cobalt titanate (labeling name, INCI name: Cobalt Titanium Oxide), fired titanium/titanium oxide (labeling name, INCI name: Titanium/-Titanium Dioxide), Li/Co titanate (labeling name, INCI name: Lithium Cobalt Titanate), cobalt titanate (labeling name, INCI name: Cobalt Titanium Oxide), sintered iron oxide/titanium oxide (labeling name, INCI name: Iron Oxide/Titanium Dioxide Sinter), composites doped with hetero-metals such as iron oxide-doped titanium oxide (labeling name, INCI name: Iron Oxides, Titanium Dioxide), titanium nitride (labeling name, INCI name: Titanium Nitride), ferrous hydroxide (labeling name, INCI name: Iron Hydroxide), inorganic brown pigments such as $\gamma$-iron oxide, inorganic yellow pigments such as ochre, and colored pigments such as lake forms of tar-based dyes and lake forms of naturally occurring dyes.

As the coloring pigment, pigments having a particle size, i.e., volume average particle size in the range of 150 to 600 nm are preferred from the aspect of covering power. The volume average particle size may be measured by TEM or the like. If the particle size is less than 150 nm, the coloring efficiency of cosmetics may be reduced due to a low covering power. If the particle size exceeds 600 nm, the feeling on use may be aggravated. The pigment used herein may have been treated on its surface in part or entirety with inorganic compounds such as alumina (labeling name, INCI name: Alumina), aluminum hydroxide (labeling name, INCI name: Aluminum Hydroxide), silica (labeling name, INCI name: Silica), and hydrated silica (labeling name, INCI name: Hydrated Silica).

Examples of the inorganic powder include microparticulate forms of zirconium oxide (labeling name, INCI name: Zirconium Dioxide), zinc oxide (labeling name, INCI name: Zinc Oxide), cerium oxide (labeling name, INCI name: Cerium Oxide), magnesium oxide (labeling name, INCI name: Magnesium Oxide), barium sulfate (labeling name, INCI name: Barium sulfate), calcium sulfate (labeling name, INCI name: Calcium Sulfate), magnesium sulfate (labeling name, INCI name: Magnesium Sulfate), calcium carbonate (labeling name, INCI name: Calcium Carbonate), magnesium carbonate (labeling name, INCI name: Magnesium Carbonate), talc (labeling name, INCI name: Talc), cleaved talc (labeling name, INCI name: Talc), mica (labeling name, INCI name: Mica), kaolin (labeling name, INCI name: Kaolin), sericite (labeling name, INCI name: Sericite), synthetic fluorphlogopite (labeling name, INCI name: Synthetic Fluorphlogopite), black mica (labeling name, INCI name: Biotite), potassium silicate (labeling name, INCI name: Potassium Silicate), silica (labeling name, INCI name: Silica), fumed silica, aluminum silicate (labeling name, INCI name: Aluminum Silicate), magnesium silicate (labeling name, INCI name: Magnesium Silicate), Al/Mg silicate (labeling name, INCI name: Magnesium Aluminum Silicate), calcium silicate (labeling name, INCI name: Calcium Silicate), Al/Ca/Na silicate (labeling name, INCI name: Aluminum Calcium Sodium Silicate), Li/Mg/Na silicate (labeling name, INCI name: Lithium Magnesium Sodium Silicate), Na/Mg silicate (labeling name, INCI name: Sodium Magnesium Silicate), Ca/Al borosilicate (labeling name, INCI name: Calcium Aluminum Borosilicate), Ca/Na borosilicate (labeling name, INCI name: Calcium Sodium Borosilicate), hydroxyapatite (labeling name, INCI name: Hydroxyapatite), bentonite (labeling name, INCI name: Bentonite), montmorillonite (labeling name, INCI name: Montmorillonite), hectorite (labeling name, INCI name: Hectorite), zeolite (labeling name, INCI name: Zeolite), alumina (labeling name, INCI name: Alumina), aluminum hydroxide (labeling name, INCI name: Aluminum Hydroxide), boron nitride (labeling name, INCI name: Boron Nitride), and glass (labeling name, INCI name: Glass). Examples of the inorganic coloring pearlescent pigment include pearlescent pigments such as titanium oxide-coated mica, bismuth oxychloride (labeling name, INCI name: Bismuth Oxychloride), bismuth oxychloride (labeling name, INCI name: Bismuth Oxychloride) coated with titanium oxide (labeling name, INCI name: Titanium Dioxide), talc (labeling name, INCI name: Talc) coated with titanium oxide (labeling name, INCI name: Titanium Dioxide), fish scale flakes, and coloring mica coated with titanium oxide (labeling name, INCI name: Titanium Dioxide).

Examples of the metal powder include microparticulate metals such as aluminum (labeling name, INCI name: Aluminum Powder), copper (labeling name, INCI name: Copper Powder), and silver (labeling name, INCI name: Silver

Powder).

Examples of the organic powder include powders of silicone, polyamide, polyacrylic acid/acrylic acid esters, polyester, polyethylene, polypropylene, polystyrene, styrene-acrylic acid copolymers, divinylbenzene-styrene copolymers, polyurethane, vinyl resins, urea resins, melamine resins, benzoguanamine, polymethylbenzoguanamine, tetrafluoroethylene, polymethyl methacrylate (e.g., polymethyl methacrylate), cellulose, silk, nylon, phenolic resins, epoxy resins, and polycarbonate. Specific examples of the silicone include silicone resin particles such as polymethylsilsesquioxane (labeling name, INCI name: polymethylsilsesquioxane), silicone resin-coated silicone rubber powder (e.g., vinyl dimethicone/methicone silsesquioxane crosspolymer (labeling name, INCI name: Vinyl Dimethicone/Methicone Silsesquioxane Crosspolymer), diphenyl dimethicone/vinyldiphenyl dimethicone/silsesquioxane crosspolymer (labeling name, INCI name: Diphenyl Dimethicone/Vinyl Diphenyl Dimethicone/Silsesquioxane Crosspolymer), polysilicone-1 crosspolymer (labeling name, INCI name: Polysilicone-1 Crosspolymer), and polysilicone-22 (labeling name, INCI name: Polysilicone-22). Also included are metal soaps, examples of which include powders of zinc stearate (labeling name, INCI name: Zinc Stearate), aluminum stearate (labeling name, INCI name: Aluminum Stearate), calcium stearate (labeling name, INCI name: Calcium Stearate), magnesium stearate (labeling name, INCI name: Magnesium Stearate), zinc myristate (labeling name, INCI name: Zinc Myristate), magnesium myristate (labeling name, INCI name: Magnesium Myristate), Zn/Na cetylphosphate (labeling name, INCI name: Sodium Zinc Cetyl Phosphate), and potassium cetylphosphate (labeling name, INCI name: Potassium Cetyl Phosphate). Also included are organic dyes, examples of which include tar dyes such as Red #3, Red #104(1) (labeling name, INCI name: Red 28, Red 28 Lake), Red #106, Red #201 (labeling name, INCI name: Red 6), Red #202 (labeling name, INCI name: Red 7 ), Red #204, Red #205, Red #220 (labeling name, INCI name: Red 34), Red #226 (labeling name, INCI name: Red 30), Red #227 (labeling name, INCI name: Red 33, Red 33 Lake), Red #228 (labeling name, INCI name: Red 36), Red #230(1) (labeling name, INCI name: Red 22, Red 22 Lake), Red #230(2), Red #401, Red #505, Yellow #4 (labeling name, INCI name: Yellow 5), Yellow #5 (labeling name, INCI name: Yellow 6, Yellow 6 Lake), Yellow #202(1) (labeling name, INCI name: Yellow 8), Yellow #203 (labeling name, INCI name: Yellow 10, Yellow 10 Lake), Yellow #204 (labeling name, INCI name: Yellow 11), Yellow #401, Blue #1 (labeling name, INCI name: Blue 1, Blue 1 Lake), Blue #2, Blue #201, Blue #205 (labeling name, INCI name: Blue 4), Blue #404, Green #3 (labeling name, INCI name: Green 3, Green 3 Lake), Green #201 (labeling name, INCI name: Green 5), Green #202 (labeling name, INCI name: Green 6), Green #204 (labeling name, INCI name: Green 8), Green #205, Orange #201 (labeling name, INCI name: Orange 5), Orange #203 (labeling name, INCI name: Pigment Orange 5), Orange #204, Orange #205 (labeling name, INCI name: Orange 4, Orange 4 Lake), Orange #206 (labeling name, INCI name: Orange 10), and Orange 207 (labeling name, INCI name: Orange 11); and naturally occurring dyes such as cochineal (labeling name, INCI name: Cochineal), laccaic acid (labeling name, INCI name: Laccaic Acid), Safflower red (labeling name, INCI name: Carthamus Tinctorius (Safflower) Flower Extract), purple root extract (labeling name, INCI name: Lithospermum Officinale Root Extract), gardenia yellow, and gardenia blue (labeling name, INCI name: Hydrolyzed Gardenia Floride Extract).

Examples of the inorganic-organic composite powder include composite powders of inorganic powders coated on their surface with organic powders by any prior art well-known methods.

The hydrophobic treating agent with which the source powder is treated on the surface is not particularly limited as long as it can impart hydrophobic properties. Suitable hydrophobic treating agents include silicone treating agents, waxes, paraffins, organic fluorine compounds consisting of perfluoroalkyls and phosphoric acid salts, surfactants, amino acids such as N-acylglutamic acid, and metal soaps such as aluminum stearate (labeling name, INCI: Aluminum Stearate) and magnesium myristate (labeling name, INCI: Magnesium Myristate).

Inter alia, silicone treating agents are preferred, examples of which include silanes or silylating agents such as triethoxycaprylylsilane (labeling name, INCI name: Triethoxycaprylylsilane), or trimethoxysilyl dimethicone (labeling name, INCI name: Trimethoxysilyl Dimethicone); silicone oils such as dimethicone (labeling name, INCI name: Dimethicone), hydrogen dimethicone (labeling name, INCI name: Hydrogen Dimethicone), triethoxysilylethyl polydimethylsiloxyethyl hexyl dimethicone (labeling name, INCI name: Triethoxysilylethyl Polydimethylsiloxyethyl Hexyl Dimethicone); and silicone compounds such as acrylate/tridecyl acrylate/triethoxysilylpropyl methacrylate/dimethicone methacrylate copolymers (labeling name, INCI name: Acrylates/Tridecyl Acrylate/Triethoxysilylpropyl Methacrylate/Dimethicone Methacrylate Copolymer) and acrylate/dimethicone copolymers (labeling name, INCI name: Acrylates/Dimethicone Copolymer). Notably, the surface hydrophobic treating agents may be used alone or in admixture.

In the practice of the invention, the method of surface treating a source powder with a hydrophobic treating agent is not particularly limited, and any well-known methods may be used. The surface treating method is generally divided into dry and wet methods. In the dry method, treatment is carried out by mixing and contacting the source powder and the hydrophobic treating agent on a suitable means, for example, stirrers, grinders, mixers, and dispersing machines such as Henschel mixers, ball mills, jet mills, kneaders, planetary mixers, sand mills, attritors, ribbon blenders, dispersing mixers, and homo-mixers. The treatment may be carried out while imparting thermal or mechano-chemical forms of energy, for example, heat, mechanical forces and overheated steam. It is also acceptable that after the source powder and the hydrophobic treating agent are thoroughly mixed and contacted, thermal or mechano-chemical forms of energy, for

example, heat, mechanical forces and overheated steam is separately imparted to carry out the treatment. Alternatively, when the hydrophobic treating agent is mixed and contacted with the source powder, the method including the steps of previously dissolving or dispersing the hydrophobic treating agent in any desired amount of water, solvents or super-critical fluids and spraying the solution or dispersion to the source powder may be employed. In the wet method, treatment may be carried out by dispersing the source powder and the hydrophobic treating agent in water, solvent or super-critical fluid, mixing and contacting, then evaporating off the solvent, and further separately imparting thermal or mechano-chemical forms of energy, for example, heat, mechanical forces and overheated steam. Examples of the hydrophobic powder are shown below, but not limited thereto.

As the microparticulate metal oxides having undergone hydrophobic treatment, any commercially available substances may be used. Examples of microparticulate titanium oxide include those marketed under the trade name of STR-100C-LP, STR-100A-LP, STR-100W, STR-100W-LP, STR-100C-LF, and STR-40-LP (Sakai Chemical Co., Ltd.), MT-01, MT-05, MT-100Z, MT-100TV, MT-100AQ, MT-150EX, MT-500B, MT-505SAS, MT-700B, MT-014Z, and SMT-500SAS (Tayca Corp.), ST-455, ST-455WS, ST-457ECS, and ST-495M (Titan Kogyo, Ltd.). Examples of microparticulate zinc oxide include those marketed under the trade name of FINEX-50S-LP2, FINEX-30S-LP2, FINEX-50W, FINEX-30W, FINEX-50W-LP2, FINEX-52W-LP2, FINEX-30W-LP2, FINEX-33W-LP2, FINEX-50-LPT, FINEX-25-LPT, FINEX-50S-LPT, and FINEX-30S-LPT (Sakai Chemical Co., Ltd.), MZ-150, MZ-200, MZ-300, MZ-306X, MZ-500HP, MZ-505T, MZX-506X, MZY-203S, MZY-210M3S, TMZ-HA1, and MZX-5080TS (Tayca Corp.)

Examples of the coloring pigment having undergone hydrophobic treatment include KTP-09 series, e.g., KTP-09W, KTP-09R, KTP-09Y, and KTP-09B (Shin-Etsu Chemical Co., Ltd.).

[Dispersion]

A further embodiment of the invention is a dispersion comprising the (treated) hydrophobic powder which is treated with (A) the organopolysiloxane. The organopolysiloxane containing (poly)glycerin and polyoxyalkylene groups as component (A) acts as a dispersant for dispersing the hydrophobic powder (B) in water (C) in a stable manner.

The dispersion contains the (treated) hydrophobic powder which is treated with (A) the organopolysiloxane. Preferred is a dispersion comprising the following components:

(A) organopolysiloxane,
(B) hydrophobic powder, and
(C) water.

The organopolysiloxane as component (A) may be used alone or in admixture of two or more. The amount of the organopolysiloxane (A) blended, though not particularly limited, is preferably 1 to 30% by weight, more preferably 5 to 11% by weight of the overall dispersion.

The amount of the hydrophobic powder (B) blended, though not particularly limited, is preferably 1 to 70% by weight, more preferably 10 to 60% by weight, even more preferably 30 to 60% by weight of the overall dispersion.

[Component (C)]

In the dispersion, water forms a water phase in which the hydrophobic powder (B) is uniformly dispersed. Examples of the water used herein include purified water commonly used in cosmetics, distilled water from fruits and plants, sea water (INCI name: Sea Water), spring water (or Onsen-Sui), and peat water (INCI name: Peat Water).

The amount of the water (C) blended, though not particularly limited, is preferably 5 to 95% by weight, more preferably 10 to 50% by weight of the overall dispersion.

[Component (D)]

In addition to the foregoing components (A) to (C), the dispersion may contain at least one polyhydric alcohol. The polyhydric alcohol, if blended, allows the organopolysiloxane (A) to uniformly distribute on the surface of hydrophobic powder (B) so that the powder is unlikely to agglomerate together and the dispersion is more improved in stability with time.

Examples of the polyhydric alcohol include ethylene glycol, propylene glycol, dipropylene glycol, 1,3-butylene glycol, pentylene glycol, glycerin, diglycerin, triglycerin, and polyglycerin. Inter alia, 1,3-butylene glycol, propylene glycol, diglycerin, glycerin and a mixture thereof are preferred.

When used, the amount of component (D) blended, though not particularly limited, is preferably 1 to 50% by weight, more preferably 1 to 25% by weight of the overall dispersion.

Any optional components to be described later may be blended in the dispersion as long as the benefits of the invention are not adversely affected. The amount of components other than components (A) to (D) is preferably up to 50%

by weight, more preferably up to 30% by weight of the overall dispersion. The benefits of the invention are obtained without blending a water-swelling substance or water-soluble polymer.

The dispersion preferably has a viscosity of 100 to 10,000 mPa s, more preferably 500 to 4,000 mPa·s as measured at 25°C by a rotational viscometer according to JIS K 7117-1:1999. The dispersion of the invention has a low viscosity and improved stability of viscosity with the lapse of time.

The method of preparing the dispersion according to the invention is not particularly limited. The dispersion can be prepared by mixing the foregoing components. The preferred method includes the steps of mixing the organopolysiloxane (A) with the hydrophobic powder (B) and dispersing the mixture in component (C). The mixing step is not particularly limited and any well-known techniques capable of uniform mixing may be employed. Any of stirrers, grinders, mixers, and dispersing machines such as Henschel mixers, ball mills, kneaders, planetary mixers, ribbon blenders, dispersing mixers, homo-mixers, roll mills and bead mills may be used.

In the dispersion, the hydrophobic powder is effectively dispersed in the water phase. For this reason, the dispersion keeps a low viscosity even when a large amount of the hydrophobic powder is blended, the hydrophobic powder is unlikely to agglomerate together, and the dispersion does not build up its viscosity with the lapse of time. The dispersion may be used by itself as a cosmetic composition. Since the continuous phase is water, the dispersion gives a fresh and/or light feeling on use when applied to the skin. A cosmetic film formed on the skin has improved water resistance because the hydrophobic powder is blended therein. Since the hydrophobic powder is satisfactorily dispersed, the cosmetic film has improved uniformity and gives a pleasant feeling on use. In addition, the dispersion is useful as a raw material for various products, especially a raw material for cosmetics, paints, and inks.

[Cosmetics]

Now the cosmetic composition is described in further detail.

Although the organopolysiloxane (A) may be blended as such, it is preferably blended in the form of the (treated) hydrophobic powder treated with the organopolysiloxane (A). Further, the organopolysiloxane (A) may be blended in the form of the dispersion. Although the amount of each component in the overall cosmetic composition is not particularly limited, the preferred amount of a component, if blended, is as follows.

The amount of the organopolysiloxane as component (A), though not particularly limited, is preferably 0.1 to 10% by weight, more preferably 0.1 to 5% by weight of the overall cosmetic composition.

The amount of the hydrophobic powder as component (B), though not particularly limited, is preferably 1 to 70% by weight, more preferably 10 to 60% by weight, even more preferably 30 to 60% by weight of the overall cosmetic composition.

The amount of water as component (C), though not particularly limited, is preferably 5 to 95% by weight, more preferably 10 to 50% by weight of the overall cosmetic composition.

If blended, the amount of component (D), though not particularly limited, is preferably 1 to 50% by weight, more preferably 1 to 25% by weight of the overall cosmetic composition.

The amount of the dispersant, though not particularly limited, is preferably 0.1 to 70% by weight, more preferably 1 to 50% by weight of the overall cosmetic composition.

[Oil]

The cosmetic composition may further contain (E) an oil. The oil may be liquid, solid, or semi-solid and selected from, for example, hydrocarbon oils, higher fatty acids, higher alcohols, esters, silicone oils, and fluorochemical oils. These oils may be used alone or in admixture.

The hydrocarbon oils include straight or branched hydrocarbon oils while they may be either volatile or nonvolatile. Examples of the hydrocarbon oil include olefin oligomers, isododecane (labeling name, INCI: Isododecane), dodecane (labeling name, INCI: Dodecane), isohexadecane (labeling name, INCI: Isohexadecane), undecane (labeling name, INCI: Undecane), squalane (labeling name, INCI: Squalane), squalene (labeling name, INCI: Squalene), mineral oil (labeling name, INCI: Mineral Oil), fluid isoparaffin, polyisobutylene (labeling name), hydrogenated polyisobutene (labeling name, INCI: Hydrogenated Polyisobutene), and (C13-15) alkane (labeling name, INCI: C13-15 Alkane).

Examples of the higher fatty acid include oleic acid (labeling name, INCI: Oleic Acid), linoleic acid (labeling name, INCI: Linoleic Acid), linolenic acid (labeling name, INCI: Linolenic Acid), arachidonic acid (labeling name, INCI: Arachidonic Acid), eicosapentaenoic acid (EPA) (labeling name, INCI: Eicosapentaenoic Acid), docosahexaenoic acid (labeling name, INCI: Docosahexaenoic Acid), isostearic acid (labeling name, INCI: Isostearic Acid), and hydroxystearic acid (labeling name, INCI: Hydroxystearic Acid).

Examples of the natural animal and plant oils and fats and semi-synthetic oils and fats include avocado oil (labeling name, INCI: Persea Gratissima (Avocado) Oil), linseed oil (labeling name, INCI: Linum Usitatissimum (Linseed) Seed Oil), almond oil (labeling name, INCI: Prunus Amygdalus Dulcis (Sweet Almond) Oil), olive oil (labeling name, INCI:

Olea Europaea (Olive) Fruit Oil), Torreya California oil (labeling name, INCI: Torreya Californica (California Nutmeg) Oil), citronella grass oil (labeling name, INCI: Cymbopogon Nardus (Citronella) Oil), shark liver oil (labeling name, INCI: Shark Liver Oil), cod liver oil (labeling name, INCI: Cod Liver Oil), fish liver oil (labeling name, INCI: Fish Liver Oil), kyounin oil (labeling name, INCI: Kyounin Yu), sesame oil (labeling name, INCI: Sesamum Indicum (Sesame) Seed Oil), rice germ oil (labeling name, INCI: Oryza Sative (Rice) Germ Oil), rice bran oil (labeling name, INCI: Oryza Sativa (Rice) Bran Oil), camellia oil (labeling name, INCI: Camellia Kissi Seed Oil), safflower oil (labeling name, INCI: Carthamus Tinctorius (Safflower) Seed Oil), turtle oil (labeling name, INCI: Turtle Oil), Japanese camellia oil (labeling name, INCI: Camellia Japonica Seed Oil), primrose oil (labeling name, INCI: Oenothera Biennis (Evening Primrose) Oil), corn germ oil (labeling name, INCI: Zea Mays (Corn) Germ Oil), rapeseed oil (labeling name, INCI: Rape Shushi Yu), wheat germ oil (labeling name, INCI: Triticum Vulgare (Wheat) Germ Oil), persic oil (labeling name), palm oil (labeling name, INCI: Elaeis Guineensis (Palm) Oil), palm kernel oil (labeling name, INCI: Elaeis Guineensis (Palm) Kernel Oil), castor oil (labeling name, INCI: Ricinus Communis (Castor) Seed Oil), hydrogenated castor oil (labeling name), sunflower oil (labeling name, INCI: Helianthus Annuus (Sunflower) Seed Oil), grape seed oil (labeling name, INCI: Vitis Vinifera (Grape) Seed Oil), jojoba seed oil (labeling name, INCI: Simmondsia Chinensis (Jojoba) Seed Oil), macadamia nut oil (labeling name, INCI: Macadamia Ternifolia Seed Oil), mink oil (labeling name, INCI: Mink Oil), meadowfoam oil (labeling name, INCI: Limnanthes Alba (Meadowfoam) Seed Oil), cotton seed oil (labeling name, INCI: Gossypium Herbaceum (Cotton) Seed Oil), coconut oil (labeling name, INCI: Cocos Nucifera (Coconut) Oil), hydrogenated coconut oil (labeling name, INCI: Hydrogenated Coconut Oil), and egg oil (labeling name, INCI: Egg Oil).

The ester oils are liquid oils in condensed forms of fatty acids of 1 to 20 carbon atoms with alcohols of 1 to 20 carbon atoms and may be monoesters, or polyesters such as diesters and triesters. Examples include diisobutyl adipate (labeling name, INCI: Diisobutyl Adipate), dihexyldecyl adipate (labeling name), diheptylundecyl adipate (labeling name, INCI: Diheptylundecyl Adipate), n-alkyl glycol monoisostearates such as isostearyl isostearate (labeling name, INCI: Isostearyl Isostearate), isocetyl isostearate (labeling name, INCI: Isocetyl Isostearate), trimethylolpropane triisostearate (labeling name, INCI: Trimethylolpropane Triisostearate), glycol diethylhexanoate (labeling name, INCI: Glycol Diethylhexanoate), cetyl ethylhexanoate (labeling name, INCI: Cetyl Ethylhexanoate), triethylhexanoin (labeling name, INCI: Triethylhexanoin), trimethylolpropane triethylhexanoate (labeling name, INCI: Trimethylolpropane Triethylhexanoate), pentaerythrityl tetraethylhexanoate (labeling name, INCI: Pentaerythrityl Tetraethylhexanoate), cetyl octanoate (labeling name, INCI: Cetyl Ethylhexanoate), octyldodecyl esters such as octyldodecyl stearoyloxystearate (labeling name, INCI: Octyldodecyl Stearoyl Stearate), oleyl oleate (labeling name, INCI: Oleyl Oleate), octyldodecyl oleate (labeling name, INCI: Octyldodecyl Oleate), decyl oleate (labeling name, INCI: Decyl Oleate), neopentyl glycol dioctanoate (labeling name, INCI: Neopentyl Glycol Diethylhexanoate), neopentyl glycol dicaprate (labeling name, INCI: Neopentyl Glycol Dicaprate), triethyl citrate (labeling name, INCI: Triethyl Citrate), diethylhexyl succinate (labeling name, INCI: Diethylhexyl Succinate), amyl acetate (labeling name, INCI: Amyl Acetate), ethyl acetate (labeling name, INCI: Ethyl Acetate), butyl acetate (labeling name, INCI: Butyl Acetate), isocetyl stearate (labeling name, INCI: Isocetyl Stearate), butyl stearate (labeling name, INCI: Butyl Stearate), diisopropyl sebacate (labeling name, INCI: Diisopropyl Sebacate), diethylhexyl sebacate (labeling name, INCI: Diethylhexyl Sebacate), cetyl lactate (labeling name, INCI: Cetyl Lactate), myristyl lactate (labeling name, INCI: Myristyl Lactate), isononyl isononanoate (labeling name, INCI: Isononyl Isononanoate), isotridecyl isononanoate (labeling name, INCI: Isotridecyl Isononanoate), palmitates such as isopropyl palmitate (labeling name, INCI: Isopropyl Palmitate), ethylhexyl palmitate (labeling name, INCI: Ethylhexyl Isopalmitate), and hexyldecyl palmitate (labeling name, INCI: Isocetyl Palmitate, Hexyldecyl Palmitate), cholesteryl hydroxystearate (labeling name, INCI: Cholesteryl Hydroxystearate), myristates such as isopropyl myristate (labeling name, INCI: Isopropyl Myristate), octyldodecyl myristate (labeling name, INCI: Octyldodecyl Myristate), and myristyl myristate (labeling name, INCI: Myristyl Myristate), ethylhexyl laurate (labeling name, INCI: Ethylhexyl Laurate), hexyl laurate (labeling name, INCI: Hexyl Laurate), dioctyldodecyl lauroyl glutamate (labeling name, INCI: Dioctyldodecyl Lauroyl Glutamate), isopropyl lauroyl sarcosinate (labeling name, INCI: Isopropyl Lauroyl Sarcosinate), diisostearyl malate (labeling name, INCI: Diisostearyl Malate), and glyceride oils such as glyceryl acetate (labeling name, INCI: Glyceryl Acetate), glyceryl stearate (labeling name, INCI: Glyceryl Stearate), and tri(caprylic/capric acid) glyceride (labeling name, INCI: Caprylic/Capric Triglyceride).

Examples of the silicone oil include straight or branched dimethicones having a low to high viscosity such as trisiloxane (labeling name, INCI: Trisiloxane), volatile dimethicone (labeling name, INCI: Dimethicone), low viscosity dimethicone (labeling name, INCI: Dimethicone), cyclotetrasiloxane (labeling name, INCI: Cyclotetrasiloxane), cyclopentasiloxane (labeling name, INCI: Cyclopentasiloxane), cyclohexasiloxane (labeling name, INCI: Cyclohexasiloxane), methyl trimethicone (labeling name, INCI: Methyl Trimethicone), caprylyl methicone (labeling name, INCI: Caprylyl Methicone), phenyl trimethicone (labeling name, INCI: Phenyl Trimethicone), methylphenylpolysiloxane (labeling name, INCI: Diphenyl Dimethicone), diphenylsiloxyphenyl trimethicone (labeling name, INCI: Diphenylsiloxy Phenyl Trimethicone), ethyl methicone (labeling name, INCI: Ethyl Methicone), ethyl trisiloxane (labeling name, INCI: Ethyl Trisiloxane), and hydrogen dimethicone (labeling name, INCI: Hydrogen Dimethicone); amodimethicone (labeling name, INCI: Amodimethicone), aminopropyl dimethicone (labeling name, INCI: Aminopropyl Dimethicone), high degree-of-polymerization gum-like dimethicone (labeling name, INCI: Dimethicone), gum-like amodimethicone (labeling name, INCI:

Amodimethicone), silicone rubbers such as gum-like dimethylsiloxane-methylphenylsiloxane copolymers, cyclic organopolysiloxane solutions of silicone gums and rubbers, amino acid-modified silicones, fluorine-modified silicones, silicone resins, silicone resin solutions.

Examples of the fluorochemical oil include perfluoropolyethers such as polyperfluoromethyl isopropyl ether (labeling name, INCI: Polyperfluoromethylisopropyl Ether), and perfluorocarbons such as perfluorodecalin (labeling name, INCI: Perfluorodecalin) and perfluorohexane (labeling name, INCI: Perfluorohexane).

When used, the amount of component (E) blended, though not particularly limited, is preferably 1 to 50% by weight, more preferably 1 to 25% by weight of the overall cosmetic composition.

[Optional components]

In the cosmetic composition of the invention, any of optional components which are commonly used in the cosmetic field may be additionally blended. Examples of the optional components include UV absorbers, surfactants other than component (A), film-forming agents, antiperspirant, antibacterial agents, preservatives, fragrances, salts, antioxidants, humectants, acidity regulators, chelating agents, refreshing agents, anti-inflammatory agents, skin modifying ingredients (e.g., brightening or whitening agents, cell activators, anti-skin-roughening agents, blood flow enhancing agents, skin astringents, antiseborrheic agents), vitamins, amino acids, nucleic acids, hormones, clathrate compounds, powders other than component (B), organic resins, and thickeners. They may be used alone or in admixture.

The UV absorber used herein is not particularly limited as long as it is commonly blended in cosmetics. The absorber may be used alone or in admixture. Examples of the oil-soluble UV absorber include homosalate (Japanese labeling name, INCI: Homosalate), octocrylene (labeling name, INCI: Octocrylene), t-butyl methoxydibenzoylmethane (labeling name, INCI: Butyl Methoxydibenzoylmethane), ethylhexyl salicylate (labeling name, INCI: Ethylhexyl Salicylate), diethylaminohydroxybenzoyl hexyl benzoate (labeling name, INCI: Diethylamino Hydroxybenzoyl Hexyl Benzoate), oxybenzone-6 (labeling name, INCI: Benzophenone-6), oxybenzone-9 (labeling name, INCI: Benzophenone-9), oxybenzone-1 (labeling name, INCI: Benzophenone-1), polysilicone-15 (labeling name, INCI: Polysilicone-15), dimethoxybenzylidene dioxoimidazolidine octyl propionate (labeling name, INCI: Ethylhexyl Dimethoxybenzylidene Dioxoimidazolidine Propionate), oxybenzone-2 (labeling name, INCI: Benzophenone-2), terephthalylidene dicamphor sulfonic acid (labeling name, INCI: Terephthalylidene Dicamphor Sulfonic Acid), ethylhexyl triazone (labeling name, INCI: Ethylhexyl Triazone), methylbis(trimethylsiloxy)silyl isopentyl trimethoxycinnamate (labeling name, INCI: Isopentyl Trimethoxycinnamate Trisiloxane), drometrizole trisiloxane (labeling name, INCI: Drometrizole Trisiloxane), dimethyl PABA ethylhexyl (labeling name, INCI: Ethylhexyl Dimethyl PABA), isopropyl para-methoxycinnamate (labeling name, INCI: Isopropyl Methoxycinnamate), ethylhexyl methoxycinnamate (labeling name, INCI: Ethylhexyl Methoxycinnamate), bisethylhexyloxyphenol methoxyphenyltriazine (labeling name, INCI: Bis-Ethylhexyloxyphenol Methoxyphenyl Triazine), oxybenzone-3 (labeling name, INCI: Benzophenone-3), oxybenzone-4 (labeling name, INCI: Benzophenone-4), oxybenzone-5 (labeling name, INCI: Benzophenone-5), phenylbenzimidazole sulfonic acid (labeling name, INCI: Phenylbenzimidazole Sulfonic Acid), methylene bisbenzotriazolyl tetramethylbutylphenol (labeling name, INCI: Methylene Bis-Benzotriazolyl Tetramethylbutylphenol), glyceryl ethylhexanoate dimethoxycinnamate (labeling name, INCI: Glyceryl Ethylhexanoate Dimethoxycinnamate), glyceryl PABA (labeling name, INCI: Glyceryl PABA), methyl diisopropylcinnamate (labeling name, INCI: Diisopropyl Methyl Cinnamate), cinoxate (labeling name, INCI: Cinoxate), ethylhexyl dimethoxybenzylidene dioxoimidazolidine propionate (labeling name, INCI: Ethylhexyl Dimethoxybenzylidene Dioxoimidazolidine Propionate), etc. Also, UVA absorbers (e.g., diethylaminohydroxybenzoyl hexyl benzoate (labeling name, INCI: Diethylamino Hydroxybenzoyl Hexyl Benzoate)) and UVB absorbers (e.g., ethylhexyl methoxycinnamate (labeling name, INCI: Ethylhexyl Methoxycinnamate)) may be used in combination. Also, each of them may be used in admixture.

Preferred are one or more oil-soluble UV absorbers which are selected from ethylhexyl methoxycinnamate (labeling name, INCI: Ethylhexyl Methoxycinnamate), diethylaminohydroxybenzoyl hexyl benzoate (labeling name, INCI: Diethylamino Hydroxybenzoyl Hexyl Benzoate), octyl salicylate (labeling name, INCI: Ethylhexyl Salicylate), polysilicone-15 (labeling name, INCI: Polysilicone-15), t-butylmethoxydibenzoylmethane (labeling name, INCI: Butyl Methoxydibenzoylmethane), oxybenzone (labeling name, INCI: Benzophenonen-6), methylene bisbenzotriazolyltetramethylbutylphenol (labeling name, INCI: Methylene Bis-Benzotriazolyl Tetramethylbutylphenol), bisethylhexyloxyphenol methoxyphenyl triazine (labeling name, INCI: Bis-Ethylhexyloxyphenol Methoxyphenyl Triazine), homosalate (labeling name, INCI: Homosalate), and octocrylene (labeling name, INCI: Octocrylene).

The surfactant other than component (A) may be nonionic, anionic, cationic or ampholytic. The surfactant is not particularly limited as long as it is commonly blended in cosmetics. Of the surfactants, preferred are partially crosslinked polyether-modified silicones, partially crosslinked polyglycerin-modified silicones, straight or branched polyoxyethylene-modified organopolysiloxanes, straight or branched polyoxyethylenepolyoxypropylene-modified organopolysiloxanes, straight or branched polyoxyethylene/alkyl-co-modified organopolysiloxanes, straight or branched polyoxyethylenepolyoxypropylene/alkyl-co-modified organopolysiloxanes, straight or branched polyglycerin-modified organopolysiloxanes, straight or branched polyglycerin/alkyl-co-modified organopolysiloxanes, and pyrrolidone-modified organopolysiloxanes.

In these surfactants, the content of hydrophilic polyoxyethylene groups, polyoxyethylenepolyoxypropylene groups or polyglycerin residues is preferably 10 to 70% by weight of the molecule. On use of partially crosslinked polyether-modified silicones or partially crosslinked polyglycerin-modified silicones, it is preferred that in a composition consisting of the crosslinked organopolysiloxane and an oil which is liquid at 25°C, the crosslinked organopolysiloxane swells with the liquid oil as a result of being impregnated with an amount of more than its own weight of the liquid oil. Suitable liquid oils used herein include liquid silicones, hydrocarbon oils, ester oils, natural animal and plant oils, semi-synthetic oils and fluorochemical oils, all included in the optional oil. Illustrative examples include low viscosity silicone having a kinematic viscosity of 0.65 to 100 $mm^2/s$ at 25°C, liquid paraffin, squalane, hydrocarbon oils such as isododecane and isohexadecane, glyceride oils such as trioctanoin, ester oils such as isotridecyl isononanoate, N-acylglutamates, and lauroylsarcosinates, and natural animal and plant oils such as macadamia nut oil. Specific examples include KSG-210, KSG-240, KSG-310, KSG-320, KSG-330, KSG-340, KSG-320Z, KSG-350Z, KSG-710, KSG-810, KSG-820, KSG-830, KSG-840, KSG-820Z, and KSG-850Z, all from Shin-Etsu Chemical Co., Ltd. Examples of the surfactant other than the crosslinked organopolysiloxane include KF-6011, KF-6013, KF-6043, KF-6017, KF-6028, KF-6038, KF-6048, KF-6100, KF-6104, KF-6105, and KF-6106, all from Shin-Etsu Chemical Co., Ltd. For any of the foregoing surfactants, the content thereof is preferably 0.1 to 20% by weight of the cosmetic composition. A surfactant content of at least 0.1% by weight enables to fully exert the function of dispersion or emulsification whereas a surfactant content of up to 20% by weight eliminates the risk that the cosmetic composition has a sticky feeling on use. For the purpose of maintaining the cosmetic composition water resistant, the surfactant preferably has a HLB value of 2 to 14.5 although the HLB is not particularly limited.

The film-forming agent is blended for the purpose of further maintaining the effect-sustainability of the cosmetic composition. A silicone based agent is preferred from the aspect of imparting water repellency though the agent is not limited thereto. Examples of the film-forming agent used herein include trimethylsiloxysilicic acid (labeling name, INCI: trimethylsiloxysilicate), acrylate silicone film-forming agent, silicone-modified norbornene, and silicone-modified pullulan. The film-forming agent may be previously dissolved in an oil which is liquid at room temperature before it is blended in the cosmetic composition. Suitable liquid oils used herein include liquid silicones, hydrocarbon oils, ester oils, natural animal and plant oils, semi-synthetic oils, and fluorochemical oils, all included in the optional oil. Illustrative examples include low viscosity silicone having a kinematic viscosity of 0.65 to 100 mm2/s at 25°C, liquid paraffin, squalane, hydrocarbon oils such as isododecane and isohexadecane, glyceride oils such as trioctanoin, ester oils such as isotridecyl isononanoate, N-acylglutamates, and lauroylsarcosinates, and natural animal and plant oils such as macadamia nut oil. Specific examples include KF-7312J which is a silicone solution of trimethylsiloxysilicic acid, KP-545 and KP-549 which are silicone solutions of acrylate silicone film-former, NBN-30-ID which is an isododecane solution of silicone-modified norbornene, TSPL-30-ID which is an isododecane solution of silicone-modified pulullan, and TSPL-30-D5 which is a solicone solution, all available from Shin-Etsu Chemical Co., Ltd.

In one embodiment of the invention wherein the cosmetic composition is a deodorant, an antiperspirant is optionally blended therein. The antiperspirant is not particularly limited as long as it astricts the skin to suppress perspiration. Any of antiperspirants of general use may be used. Examples include aluminum chlorohydrate, aluminum chloride, aluminum chlorohydroxy allantoinate, aluminum allantoinate, tannic acid, aluminum potassium sulfate, zinc oxide, zinc p-phenolsulfonate, burnt alum, aluminum zirconium tetrachlorohydrate, and aluminum zirconium trichlorohydrex glycine. The preferred component which exerts a higher effect is an antiperspirant selected from aluminum halides, aluminum hydroxyhalides, and complexes or mixtures thereof with zirconyl oxyhalides and zirconyl hydroxyhalides. On use, these antiperspirants may be dissolved in water prior to blending or blended in powder form as such. Any of commercially available antiperspirants may be used. The commercially available product may take the form of a mixture with other components. The content of antiperspirant is not particularly limited and may be changed depending on the contents of other components. The content of antiperspirant is preferably 0.001 to 30% by weight, more preferably 0.01 to 20% by weight of the cosmetic composition for the purpose of obtaining a deodorant agent having a satisfactory antiperspirant effect or obtaining a deodorant agent which is less stimulative to the skin.

The antibacterial agent is not particularly limited as long as it exerts a deodorant effect by controlling the propagation of indigenous bacteria on the skin which produce body odor-causing substances. Antibacterial agents including triclosan, benzalkonium chloride, benzethonium chloride, chlorhexidine hydrochloride, chlorhexidine gluconate, cloflucarban, and isomethylphenol are generally used. Any of extracts from green tea by dry distillation and essential oils or extracts from crude drugs may also be used as long as it has an antibacterial function. Examples of the antibacterial agent having a deodorant effect, included in the essential oils or extracts from crude drugs, include green tea extract, lavender extract, scutellaria baicalensis extract, Japanese goldthread extract, plantago asiatica extract, capillary wormwood extract, aloe extract, sophora angustifolia root extract, sasa veitchil leaf extract, garlic extract, witch-hazel extract, black tea extract, sage leaf extract, zanthoxylum piperitum peel extract, ginger root extract, acorus calamus rhizome extract, ivy extract, houttuynia cordata extract, peach fruit extract, peach leaf extract, peppermint leaf extract, cnidium officinale extract, eucalyptus leaf extract, peanut seedcoat extract, ganoderma lucidum extract, and sanguisorba officinalis root extract.

Suitable preservatives include alkyl p-hydroxybenzoates, benzoic acid, sodium benzoate, sorbic acid, potassium sorbate, phenoxyethanol. Suitable antibacterial agents include benzoic acid, salicylic acid, phenol, sorbic acid, alkyl p-hydroxybenzoates, p-chloro-m-cresol, hexachlorophene, trichlorocarbanilide, and photosensitizers.

Fragrances include natural fragrances and synthetic fragrances. Suitable natural fragrances include plant fragrances separated from flowers, leaves, stems, and pericarps, and animal fragrances such as musk and civet. Suitable synthetic fragrances include hydrocarbons such as monoterpene; alcohols such as aliphatic alcohols and aromatic alcohols; aldehydes such as terpene aldehydes and aromatic aldehydes; ketones such as alicyclic ketones; esters such as terpene esters; lactones; phenols; oxides; nitrogen-containing compounds; and acetals.

Salts include inorganic salts, organic acid salts, amine salts, and amino acid salts. Exemplary inorganic salts include sodium, potassium, magnesium, calcium, aluminum, zirconium and zinc salts of inorganic acids such as hydrochloric acid, sulfuric acid, carbonic acid and nitric acid. Exemplary organic acid salts include salts of organic acids such as acetic acid, dehydroacetic acid, citric acid, malic acid, succinic acid, ascorbic acid, and stearic acid. Exemplary amine salts and amino acid salts include salts of amines such as triethanol amine, and salts of amino acids such as glutamic acid. Also useful are hyaluronic acid, salts such as chondroitin sulfate, aluminum zirconium glycine complex, and acid-alkali neutralized salts used in cosmetic formulation.

Examples of the antioxidant include, but are not particularly limited to, carotenoid, ascorbic acid and salts thereof, ascorbyl stearate, tocopherol acetate, tocopherol, p-t-butylphenol, butyl hydroxyanisole, dibutyl hydroxytoluene, phytic acid, ferulic acid, thiotaurine, hypotaurine, sulfites, erythorbic acid and salts thereof, chlorogenic acid, epicatechin, epigallocatechin, epigallocatechin gallate, apigenin, kaempferol, myricetin, and quercetin.

Suitable humectants include glucose, xylitol, maltitol, polyethylene glycol, hyaluronic acid, chondroitin sulfate, pyrrolidone carboxylate, polyoxyethylene methyl glucoside, polyoxypropylene methyl glucoside, yolk lecithin, soybean lecithin, phosphatidylcholine, phosphatidylethanol, phosphatidylserine, phosphatidylglycerol, phosphatidylinositol, and sphingolipid.

Suitable acidity regulators include lactic acid, citric acid, glycolic acid, succinic acid, tartaric acid, dl-malic acid, potassium carbonate, sodium hydrogencarbonate, and ammonium hydrogencarbonate.

Suitable chelating agents include alanine, sodium EDTA, sodium polyphosphate, sodium metaphosphate, phosphoric acid, aspartic acid diacetate, and ethylenediamine disuccinate.

Suitable refreshing agents include L-menthol and camphor.

Suitable anti-inflammatory agents include allantoin, glycyrrhizinic acid and salts thereof, glycyrrhetinic acid, stearyl glycyrrhetinate, tranexamic acid, and azulene.

Suitable skin modifying ingredients include brightening or whitening agents such as vitamin C derivatives, hydroquinone, tranexamic acid, arbutin, phenyl ethyl resorcinol, kojic acid, plant extracts; cell activators such as royal jelly, photosensitizers, cholesterol derivatives, and bovine blood extracts; anti-skin-roughening agents; blood flow enhancing agents such as vanillylamide nonanoate, benzyl nicotinate, β-butoxyethyl nicotinate, capsaicin, zingerone, cantharides tincture, ichthammol, caffeine, tannic acid, α-borneol, tocopherol nicotinate, inositol hexanicotinate, cyclandelate, cinnarizine, tolazoline, acetylcholine, verapamil, cepharanthine, and γ-oryzanol; skin astringents such as zinc oxide and tannic acid; and antiseborrheic agents such as sulfur and thianthol.

Suitable vitamins include vitamin A family such as vitamin A oil, retinol, retinol acetate, retinol palmitate; vitamin B family, specifically vitamin B2 family such as riboflavin, riboflavin butyrate, and flavin adenine nucleotide, vitamin B6 family such as pyridoxine hydrochloride, pyridoxine dioctanoate, pyridoxine tripalmitate; vitamin B12 and derivatives thereof, vitamin B15 and derivatives thereof; vitamin C family such as L-ascorbic acid, L-ascorbic acid dipalmitic acid ester, L-ascorbic acid-2-sodium sulfate, and dipotassium L-ascorbic acid phosphoric acid diester; vitamin D family such as ergocalciferol and cholecalciferol; vitamin E family such as α-tocopherol, β-tocopherol, γ-tocopherol, dl-α-tocopherol acetate, dl-α-tocopherol nicotinate, and dl-α-tocopherol succinate; nicotinic acids such as nicotinic acid, benzyl nicotinate, and nicotinic amide; vitamin H, vitamin P, pantothenic acids such as calcium pantothenate, D-pantothenyl alcohol, pantothenyl ethyl ether, acetylpantothenyl ethyl ether; and biotin.

Suitable amino acids include glycine, valine, leucine, isoleucine, serine, threonine, phenylalanine, arginine, lysine, aspartic acid, glutamic acid, cystine, cysteine, methionine, and tryptophan.

Typical of the nucleic acid is deoxyribonucleic acid.

Suitable hormones include estradiol and ethenyl estradiol.

Typical of the clathrate compound is cyclodextrin.

When the organopolysiloxane (A) is used as an emulsifier, an emulsion composition having excellent emulsion stability attributable to the polyoxyalkylene group and moistening feeling attributable to the (poly)glycerin group is obtained.

Although the organopolysiloxane (A) may be blended in a cosmetic composition independent of the shape or form thereof, it is preferred to use the organopolysiloxane as an emulsifier for dispersing the hydrophobic powder in the water phase. The method of blending the (treated) hydrophobic powder treated with the organopolysiloxane (A) in a cosmetic composition (inclusive of the method of blending the dispersion) is more preferred. The cosmetic composition

having these components blended therein gives a fresh and/or light feeling on use when applied to the skin, and forms on the skin a cosmetic film having excellent water resistance by virtue of the hydrophobic powder blended therein. Since the organopolysiloxane (A) helps disperse the hydrophobic powder (B) in water effectively, the resulting cosmetic film is fully uniform and gives a pleasant smooth feeling on use.

The method of preparing the cosmetic composition according to the invention is not particularly limited. The cosmetic composition may be prepared by blending the dispersion or component (A) with cosmetic ingredients according to a conventional preparation procedure. The method including the step of mixing component (A) with component (B) for treatment is preferred. For example, the following methods:

method (I) including the steps of previously mixing component (A) with component (B) and blending the mixture into a water phase, and

method (II) including the steps of previously mixing components (A) to (C) and optionally, component (D) to form a dispersion, and blending the dispersion are preferred.

With respect to preferred ranges and other parameters, the step of previously mixing component (A) with component (B) in method (I) is the same as the method of preparing the treated hydrophobic powder treated with the organopolysiloxane (A). With respect to preferred ranges and other parameters, the step of previously mixing components (A) to (C) and optionally, component (D) to form a dispersion in method (II) is the same as the method of preparing the dispersion.

The inventive cosmetic composition is not particularly limited in form and may take any of forms including liquid, cream, paste, solid, moose, spray, gel and emulsion. The cosmetic composition is not particularly limited in utility and may be used as skin care, make-up, hair care, and sun-screen cosmetic products. Illustrative cosmetic products include make-up cosmetic products such as skin care, hair care, cleansing, make-up base, concealer, liquid foundation, cream foundation, solid foundation, cheek color, eye shadow, mascara, eye liner, eye brow, and lipstick, and UV-protecting cosmetic products such as sun care emulsions and creams.

The inventive cosmetic composition is not particularly limited in formula and is applicable as any of oily, aqueous, O/W, and W/O cosmetic compositions, for example, preferably in the formula of O/W cosmetic composition. The cosmetic composition of O/W formula has advantages that water in the outer phase gives a fresh and/or light feeling. As water volatilizes off, the adhesion of hydrophobic powder to the skin is improved and the composition forms a uniform cosmetic film having high water resistance. This leads to improvements in lasting quality and feeling on use.

## EXAMPLES

Examples and Comparative Examples are shown below by way of illustration and not by way of limitation. The viscosity of a composition is as measured at 25°C by a rotational viscometer according to JIS K 7117-1: 1999. In Examples, the blending amounts are the amounts of the described ingredients blended. The order of arrangement of siloxane units is not limited to the described one.

[Synthesis Example 1]

A reactor was charged with (H) methylhydrogenpolysiloxane, (ii) alkene, and (v) organopolysiloxane in the molar ratio shown in Table 1, after which a platinum catalyst was added for reaction to take place at 80°C. The reactor was then charged with (iii) (poly)glycerin monoallyl ether and (iv) polyoxyalkylene monoallyl ether in the molar ratio shown in Table 1. Further, isopropyl alcohol and a platinum catalyst were added for reaction to take place at 80°C. The isopropyl alcohol was removed by heating under reduced pressure, obtaining organopolysiloxane (A-1) having polyglycerin and polyoxyalkylene groups of Synthesis Example 1. The amount of $R^2$ was 15% by weight of the total weight of organopolysiloxane (A-1).

(H-1)

[Chem. 14]

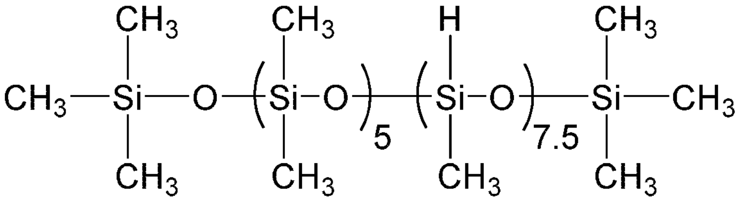

(ii-1)

[Chem. 15]

$$CH_2=CH-C_{10}H_{21}$$

(iii-1)

[Chem. 16]

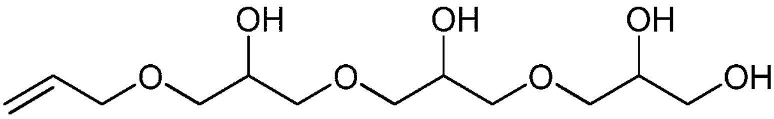

(iv-1)

[Chem. 17]

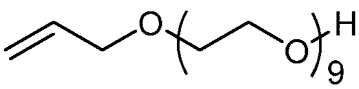

(A-1)

[Chem. 18]

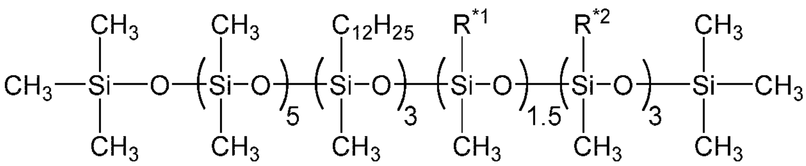

$R^{*1}$: $-C_3H_6O(CH_2CH(OH)CH_2O)_3H$

$R^{*2}$: $-C_3H_6O(C_2H_4O)_9H$

[Synthesis Example 2]

Organopolysiloxane (A-2) was obtained by the same procedure as in Synthesis Example 1 while feeding the components in the molar ratio shown in Table 1 and carrying out reaction. The amount of $R^2$ was 14% by weight of the total weight of organopolysiloxane (A-2).

(H-2)

[Chem. 19]

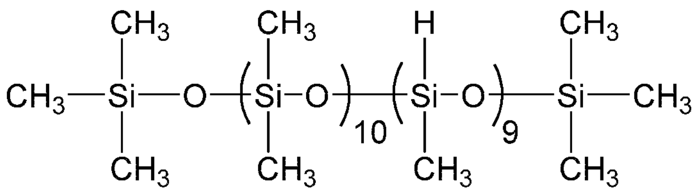

(ii-2)

[Chem. 20]

$$CH_2=CH-C_{14}H_{29}$$

(iii-1)

[Chem. 21]

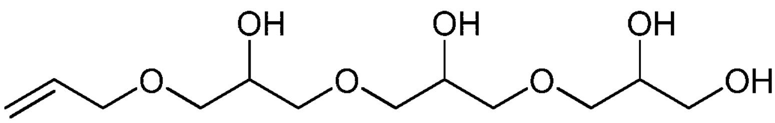

(iv-1)

[Chem. 22]

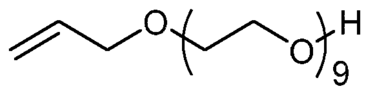

(A-2)

[Chem. 23]

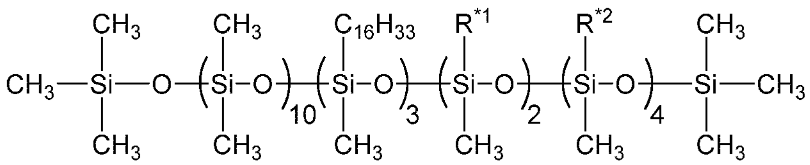

$R^{*1}$: $-C_3H_6O(CH_2CH(OH)CH_2O)_3H$
$R^{*2}$: $-C_3H_6O(C_2H_4O)_9H$

[Synthesis Example 3]

Organopolysiloxane (A-3) was obtained by the same procedure as in Synthesis Example 1 while feeding the components in the molar ratio shown in Table 1 and carrying out reaction. The amount of $R^2$ in formula (1) was 14% by weight of the total weight of organopolysiloxane (A-3).

(H-3)

[Chem. 24]

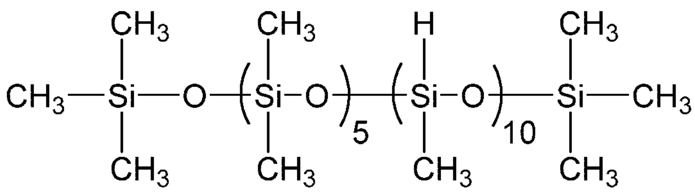

(ii-1)

[Chem. 25] $CH_2{=}CH\text{-}C_{10}H_{21}$

(iii-2)

[Chem. 26]

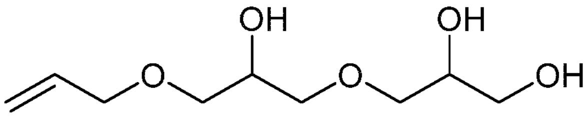

(iv-1)

[Chem. 27]

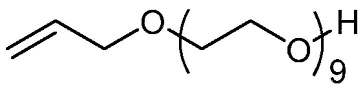

(v-1)

[Chem. 28]

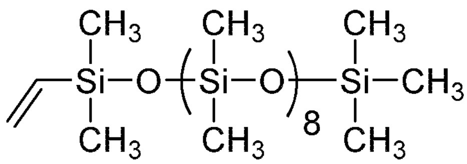

(A-3)

[Chem. 29]

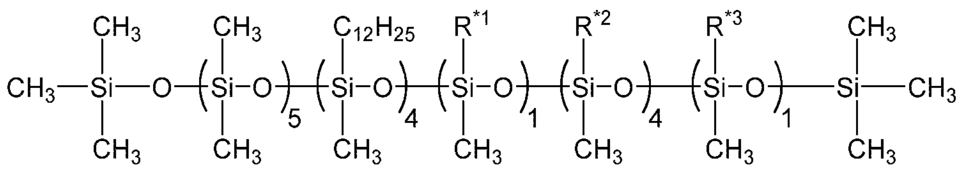

$R^{*1}$: $-C_3H_6O(CH_2CH(OH)CH_2O)_2H$

$R^{*2}$: $-C_3H_6O(C_2H_4O)_9H$

$R^{*3}$: 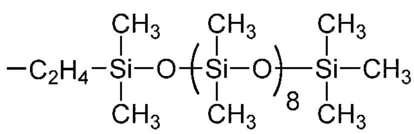

[Synthesis Example 4]

Organopolysiloxane (A-4) was obtained by the same procedure as in Synthesis Example 1 while feeding the components in the molar ratio shown in Table 1 and carrying out reaction.

(H-4)

[Chem. 30]

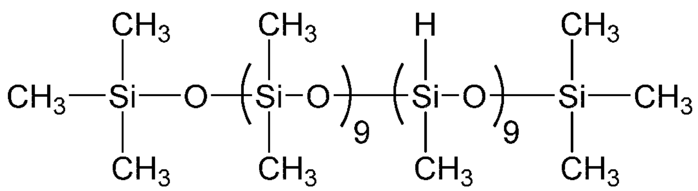

(iii-3)

[Chem. 31]

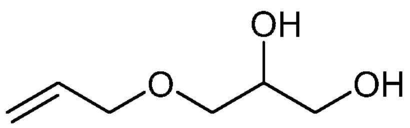

(iv-1)

[Chem. 32]

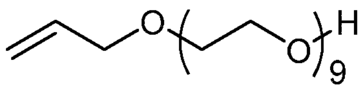

(v-2)

[Chem. 33]

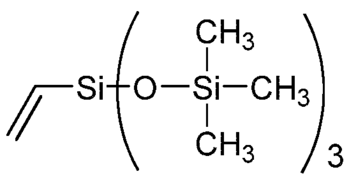

(A-4)

[Chem. 34]

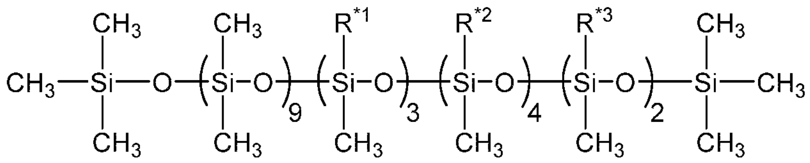

$R^{*1}$: $-C_3H_6OCH_2CH(OH)CH_2OH$
$R^{*2}$: $-C_3H_6O(C_2H_4O)_9H$

$R^{*3}$: 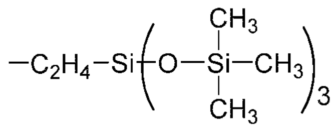

[Synthesis Example 5]

Organopolysiloxane (A-5) was obtained by the same procedure as in Synthesis Example 1 while feeding the components in the molar ratio shown in Table 1 and carrying out reaction. The amount of $R^2$ was 19% by weight of the total weight of organopolysiloxane (A-5).

(H-5)

[Chem. 35]

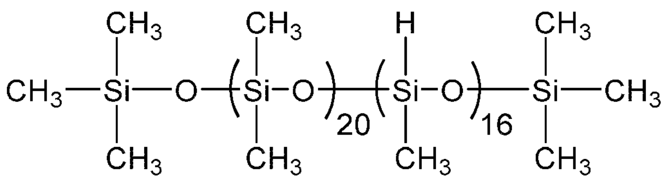

(ii-1)

[Chem. 36] $CH_2=CH-C_{10}H_{21}$

(iii-1)

[Chem. 37]

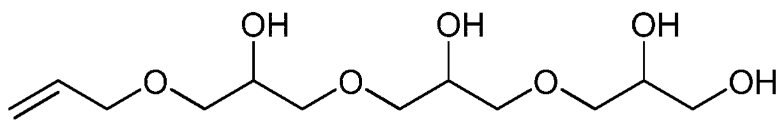

(iv-2)

[Chem. 38]

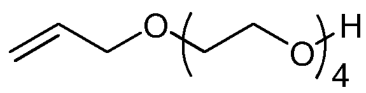

(A-5)

[Chem. 39]

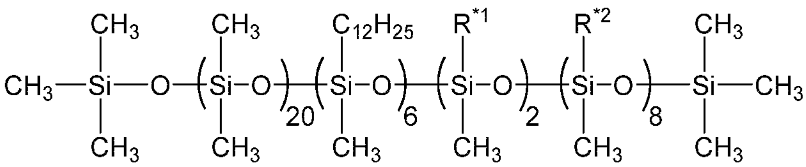

R*1: $-C_3H_6O(CH_2CH(OH)CH_2O)_3H$
R*2: $-C_3H_6O(C_2H_4O)_4H$

[Synthesis Example 6]

Organopolysiloxane (A-6) was obtained by the same procedure as in Synthesis Example 1 while feeding the components in the molar ratio shown in Table 1 and carrying out reaction.

(H-6)

[Chem. 40]

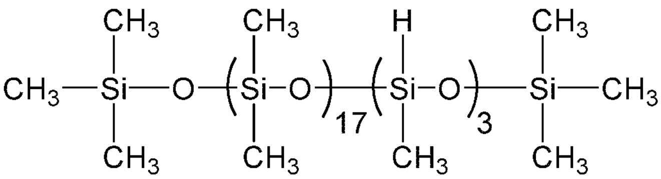

(iii-1)

[Chem. 41]

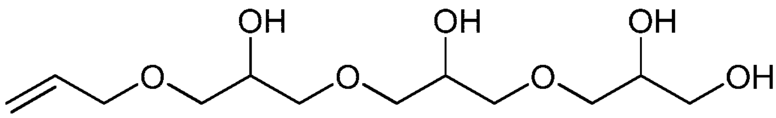

(iv-1)

[Chem. 42]

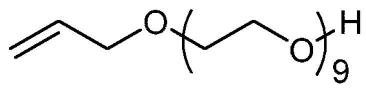

(A-6)

[Chem. 43]

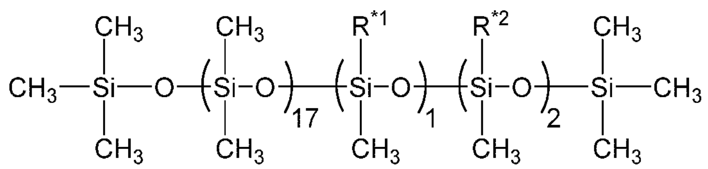

$R^{*1}$: $-C_3H_6O(CH_2CH(OH)CH_2O)_3H$

$R^{*2}$: $-C_3H_6O(C_2H_4O)_9H$

[Table 1]

| Synthesis Example | Synthesis Example 1 | Synthesis Example 2 | Synthesis Example 3 | Synthesis Example 4 | Synthesis Example 5 | Synthesis Example 6 |
|---|---|---|---|---|---|---|
| (A) Organopolysiloxane | A-1 | A-2 | A-3 | A-4 | A-5 | A-6 |
| (H) Methylhydrogenpolysiloxane | H-1 | H-2 | H-3 | H-4 | H-5 | H-6 |
| | 1 | 1 | 1 | 1 | 1 | 1 |
| (ii) Alkene | ii-1 | ii-2 | ii-1 | - | ii-1 | - |
| | 3 | 3 | 4 | 0 | 6 | 0 |
| (iii) (Poly)glycerin monoallyl ether | iii-1 | iii-1 | iii-2 | iii-3 | iii-1 | iii-1 |
| | 1.5 | 2 | 1 | 1.5 | 2 | 1 |
| (iv) Polyoxyalkylene monoallyl ether | iv-1 | iv-1 | iv-1 | iv-1 | iv-2 | iv-1 |
| | 3 | 4 | 4 | 4 | 8 | 2 |
| (v) Organopolysiloxane | - | - | v-1 | v-2 | - | - |
| | 0 | 0 | 1 | 2 | 0 | 0 |

The values of "a" and other subscripts in compositional formula (1) are shown in Table 2.

[Table 2]

| | Synthesis Example 1 | Synthesis Example 2 | Synthesis Example 3 | Synthesis Example 4 | Synthesis Example 5 | Synthesis Example 6 |
|---|---|---|---|---|---|---|
| a | 5 | 10 | 5 | 9 | 20 | 17 |
| b | 3 | 3 | 4 | 0 | 6 | 0 |
| c | 1.5 | 2 | 1 | 3 | 20 | 1 |
| d | 3 | 4 | 4 | 4 | 8 | 2 |
| e | 0 | 0 | 1 | 2 | 0 | 0 |
| (c/d) | 0.5 | 0.5 | 0.25 | 0.75 | 0.25 | 0.5 |

[Examples 1 to 6 and Comparative Examples 1 to 7]

Water dispersions of powders were obtained by mixing amounts of components as shown in Tables 3 and 4 according to the preparation procedure described below. In Tables, "Synthesis Example 1" designates organopolysiloxane (A-1) obtained in Synthesis Example 1.

[Table 3]

| Composition (wt%) | | Example 1 | Example 2 | Example 3 | Example 4 | Example 5 | Example 6 |
|---|---|---|---|---|---|---|---|
| (A) | Synthesis Example 1 | 10 | - | - | - | - | - |
| | Synthesis Example 2 | - | 10 | - | - | - | - |
| | Synthesis Example 3 | - | - | 10 | - | - | - |
| | Synthesis Example 4 | - | - | - | 10 | - | - |
| | Synthesis Example 5 | - | - | - | - | 10 | - |
| | Synthesis Example 6 | - | - | - | - | - | 10 |
| 1,3-butylene glycol | | 22 | 22 | 22 | 22 | 22 | 22 |
| Purified water | | balance | balance | balance | balance | balance | balance |
| (B) | Hydrophobized titanium oxide (*6) | 50 | - | 50 | 50 | 50 | 50 |
| | Hydrophobized titanium oxide (*7) | - | 50 | - | - | - | - |
| | Titanium oxide (*8) | - | 50 | - | - | - | - |
| Total | | 100 | 100 | 100 | 100 | 100 | 100 |
| Dispersion viscosity (mPa·s) | Immediately after preparation | 1,800 | 2,500 | 2,300 | 2,800 | 1,900 | 1,700 |
| | 1 month after preparation | 2,100 | 2,700 | 2,900 | 2,500 | 2,400 | 2,400 |
| Feeling on use | | ◎ | ◎ | ○ | ◎ | ◎ | ◎ |
| Water resistance | | ◎ | ◎ | ◎ | ○ | ◎ | ◎ |

[Table 4]

| Composition (wt%) | | | Comp. Ex. 1 | Comp. Ex. 2 | Comp. Ex. 3 | Comp. Ex. 4 | Comp. Ex. 5 | Comp. Ex. 6 | Comp. Ex. 7 |
|---|---|---|---|---|---|---|---|---|---|
| (A) | Polyether-modified silicone (*1) | | 10 | - | - | 5 | - | - | - |
| | Polyether-modified silicone (*2) | | - | 10 | - | - | - | - | - |
| | Polyglycerin-modified silicone (*3) | | - | - | 10 | 5 | - | - | - |
| | Polyglycerin-modified silicone (*4) | | - | - | - | - | 10 | - | - |
| | Polyether-modified silicone (*5) | | - | - | - | - | - | - | 10 |
| 1,3-butylene glycol | | | 22 | 22 | 22 | 22 | 22 | 22 | 22 |
| Purified water | | | balance | balance | balance | balance | balance | balance | balance |
| (B) | Hydrophobized titanium oxide (*6) | | 50 | 50 | 50 | 50 | 50 | - | 50 |
| | Hydrophobized titanium oxide (*7) | | - | - | - | - | - | - | - |
| | Titanium oxide (*8) | | - | - | - | - | - | 50 | - |
| Total | | | 100 | 100 | 100 | 100 | 100 | 100 | 100 |
| Dispersion viscosity (mPa·s) | | Immediately after preparation | 4,000 | 4,300 | 12,200 | 4,400 | 8,700 | 2,200 | 2,200 |
| | | 1 month after preparation | 5,700 | 7,000 | 18,300 | 9,100 | 15,200 | 2,600 | 2,900 |
| Feeling on use | | | Δ | Δ | ×× | × | × | ◎ | Δ |
| Water resistance | | | Δ | × | × | Δ | ×× | ×× | ◎ |

Components in Tables 5 and 6

(*1) Polyether-modified silicone: KF-6013 (Shin-Etsu Chemical Co., Ltd.)

(*2) Polyether-modified silicone: KF-6043 (Shin-Etsu Chemical Co., Ltd.)

(*3) Polyglycerin-modified silicone: KF-6105 (Shin-Etsu Chemical Co., Ltd.)

(*4) Polyglycerin-modified silicone having formula (A-7)

[Chem. 44]

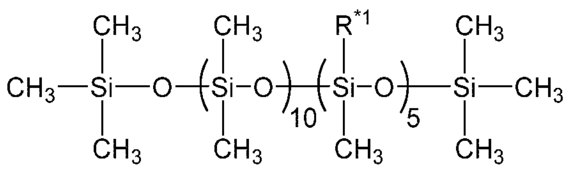

R*1: $-C_3H_6O(C_2H_4O)_9(CH_2CH(OH)CH_2O)_2H$

(*5) Polyether-modified silicone having formula (A-8)

[Chem. 45]

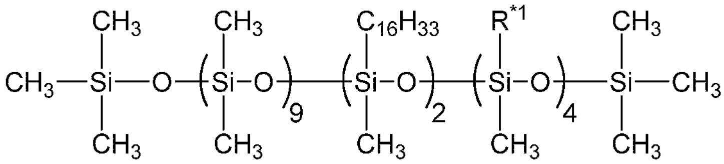

R*1: $-C_3H_6O(C_2H_4O)_9H$

(*6) Hydrophobized titanium oxide: STR-100W-OTS (Sakai Chemical Co., Ltd., treated with triethoxycaprylylsilane)

(*7) Hydrophobized titanium oxide: STR-100A-LP (Sakai Chemical Co., Ltd., treated with hydrogen dimethicone)

(*8) Titanium oxide: STR-100W (Sakai Chemical Co., Ltd., treated with hydrous silica)

(Preparation method)

A paste composition was prepared by mixing component (A), 1,3-butylene glycol, and component (B). With agitation on a dispersing mixer, water was mixed with the paste to form a water dispersion. It was confirmed that the same water dispersion as above could be prepared by mixing component (A), 1,3-butylene glycol, water, and component (B) all at once and then agitating on a dispersing mixer.

The water dispersions of Examples 1 to 6 and Comparative Examples 1 to 7 were evaluated for viscosity, stability of viscosity with time, feeling on use (fresh and smooth feeling), and water resistance by the following test methods. The results are shown in Tables 3 and 4.

[Dispersion viscosity and stability of viscosity with time]

The dispersion was stored in a 100-g glass bottle at 25°C for 3 months. The viscosity of the slurry was measured at 25°C immediately and 1 month after preparation by a Brookfield viscometer (TVB-10 type by Toki Sangyo Co., Ltd.) according to the method of JIS K 7117-1: 1999.

[Feeling on use and water resistance]

The dispersion was coated onto the back of the right hand and evaluated for feeling on use (fresh and smooth feeling) and water resistance according to the following criterion by a panel often specialists. The result was the average of ten specialists and judged according to the following criterion.

(Evaluation criterion)

[Table 5]

| Item | feeling on use (fresh and smooth) | water resistance |
|---|---|---|
| 5 points | excellent | does not ruin with sweat |
| 4 points | pretty good | - |
| 3 points | good | ruin to some extent with sweat |
| 2 points | somewhat poor | - |
| 1 point | poor | ruin with sweat |

(Judgment criterion)

| | |
|---|---|
| ◎: | 4.5 ≤ average point |
| O: | 3.5 ≤ average point < 4.5 |
| Δ: | 2.5 ≤ average point < 3.5 |
| ×: | 1.5 ≤ average point < 2.5 |
| ××: | average point < 1.5 |

As seen from the results in Tables, the water dispersions containing organopolysiloxane having (poly)glycerin and polyoxyalkylene groups in Examples 1 to 6 were satisfactory in dispersibility of hydrophobic powder, feeling on use, and water resistance. On the contrary, the water dispersions containing polyglycerin or polyether-modified organopolysiloxane in Comparative Examples 1 to 3 were poor in feeling on use and water resistance, had a high dispersion viscosity, and lacked usability. The water dispersion, which was prepared using both polyglycerin-modified silicone and polyether-modified silicone in Comparative Example 4, was poor in feeling on use and water resistance and built up its viscosity with the lapse of time. The water dispersion, which was prepared by blending polyglycerin-modified silicone in which polyglycerin is linked to the backbone silicone via polyether in Comparative Example 5, was poor in feeling on use, water resistance, and dispersibility. The water dispersion, which was prepared using hydrophilic powder in Comparative Example 6, showed good dispersibility as demonstrated by a low dispersion viscosity, but lacked water resistance. The dispersion, which was prepared by blending alkyl-containing polyether-modified silicone in Comparative Example 7, had satisfactory water resistance and a feeling on use which was pretty good, but inferior to Examples 1 to 6 in which organopolysiloxane having (poly)glycerin and polyoxyalkylene groups was blended.

[Examples 7 to 8 and Comparative Examples 8 to 10] O/W liquid foundation (cosmetic composition having hydrophobic powder in water phase)

An O/W liquid foundation was obtained by mixing components in the amounts shown in Table 6 according to the preparation method described below.

[Table 6]

| Composition (wt%) | | | Example 7 | Example 8 | Comp. Example 8 | Comp. Example 9 | Comp. Example 10 |
|---|---|---|---|---|---|---|---|
| | 1 | Stearic acid | 1.0 | 1.0 | 1.0 | 1.0 | 1.0 |
| | 2 | Behenyl alcohol | 0.4 | 0.4 | 0.4 | 0.4 | 0.4 |
| | 3 | Glyceryl stearate | 0.3 | 0.3 | 0.3 | 0.3 | 0.3 |
| | 4 | Liquid paraffin | 10.0 | 10.0 | 10.0 | 10.0 | 10.0 |
| | 5 | Tri(caprylic acid/capric acid) glyceryl | 5.0 | 5.0 | 5.0 | 5.0 | 5.0 |
| | 6 | Long-chain alkyl-containing acrylic silicone resin (*1) | 3.0 | 3.0 | 3.0 | 3.0 | 3.0 |
| | 7 | Sorbitan sesquioleate | 0.5 | 0.5 | 0.5 | 0.5 | 0.5 |
| | 8 | Sorbitan monooleate | 1.0 | 1.0 | 1.0 | 1.0 | 1.0 |
| | 9 | Acrylic-alkyl copolymer | 2.2 | 2.2 | 2.2 | 2.2 | 2.2 |
| | 10 | Triethanol amine | 1.0 | 1.0 | 1.0 | 1.0 | 1.0 |
| | 11 | Ethanol | 3.0 | 3.0 | 3.0 | 3.0 | 3.0 |
| | 12 | Hybrid silicone composite powder (*2) | 3.0 | 3.0 | 3.0 | 3.0 | 3.0 |

(continued)

| Composition (wt%) | | | Example 7 | Example 8 | Comp. Example 8 | Comp. Example 9 | Comp. Example 10 |
|---|---|---|---|---|---|---|---|
| (A) | 13 | Synthesis Example 1 | 0.2 | - | - | - | - |
| | 14 | Synthesis Example 2 | - | 0.2 | - | - | - |
| | 15 | Polyether-modified silicone (*3) | - | - | 0.2 | - | - |
| | 16 | Polyglycerin-modified silicone (*4) | - | - | - | 0.2 | - |
| | 17 | Alkyl-POE palmityl ether phosphate | 0.1 | 0.1 | 0.1 | 0.1 | 0.1 |
| | 18 | POE hydrogenated castor oil | 0.5 | 0.5 | 0.5 | 0.5 | 0.5 |
| | 19 | Hydrophobized titanium oxide (*5) | 8.5 | 8.5 | 8.5 | 8.5 | 8.5 |
| | 20 | Hydrophobized red iron oxide (*6) | 0.4 | 0.4 | 0.4 | 0.4 | 0.4 |
| | 21 | Hydrophobized yellow iron oxide (*6) | 1.0 | 1.0 | 1.0 | 1.0 | 1.0 |
| | 22 | Hydrophobized black iron oxide (*6) | 0.1 | 0.1 | 0.1 | 0.1 | 0.1 |
| | 23 | 1,3-butylene glycol | 7.0 | 7.0 | 7.0 | 7.0 | 7.2 |
| | 24 | Preservative | proper | proper | proper | proper | proper |
| | 25 | Fragrance | proper | proper | proper | proper | proper |
| | 26 | Purified water | balance | balance | balance | balance | balance |
| Total | | | 100.0 | 100.0 | 100.0 | 100.0 | 100.0 |
| Feeling on use | | | ◎ | ◎ | Δ | Δ | Δ |
| Water resistance | | | ◎ | ◎ | Δ | ○ | ×× |

(*1) Long-chain alkyl-containing acrylic silicone resin: KP-561P (Shin-Etsu Chemical Co., Ltd.)
(*2) Hybrid silicone composite powder: KSP-100 (Shin-Etsu Chemical Co., Ltd.)
(*3) Polyether-modified silicone: KF-6013 (Shin-Etsu Chemical Co., Ltd.)
(*4) Polyglycerin-modified silicone: KF-6105 (Shin-Etsu Chemical Co., Ltd.)
(*5) Hydrophobized titanium oxide: STR-100W-OTS (Sakai Chemical Co., Ltd.)
(*6) Hydrophobized iron oxide powder: treated with 2% KF-9909 (Shin-Etsu Chemical Co., Ltd.)

(Preparation method)

Step 1:
mixing components (A), (17) and (18) with a part of component (23), adding components (19) to (22) thereto, dispersing and heating
Step 2:
mixing components (1) to (8) and heating to form a solution
Step 3:
mixing components (9), (10), the remainder of (23), (24) and (26) and heating
Step 4:
mixing components (11) and (12)
Step 5:

adding the solution of Step 2 to the mixture of Step 3 with stirring, for emulsification, adding the mixture of Step 1 to the emulsion, further adding the mixture of Step 4 and component (25) thereto to yield an O/W liquid foundation

The O/W liquid foundations of Examples 7 and 8 and Comparative Examples 8 to 10 were evaluated for feeling on use (fresh and smooth feeling) and water resistance according to the same evaluation method as in Examples 1 to 6. The results are also shown in Table 6.

As seen from the results in Table 6, the O/W liquid foundations containing organopolysiloxane having (poly) glycerin and polyoxyalkylene groups in Examples 7 and 8 were satisfactory in feeling on use and water resistance. They showed a fine texture, satisfactory stability with time, and smooth spread on the skin, and gave a light feeling on use. Cosmetic films obtained from the O/W liquid foundations of Examples 7 and 8 showed satisfactory uniformity, water resistance, cold proofing, and long-lasting properties. On the contrary, the O/W liquid foundations, which were obtained by blending polyglycerin or polyoxyalkylene-modified organopolysiloxane in Comparative Examples 8 and 9, were insufficient in dispersion of hydrophobic powder and showed inferior feeling on use and water resistance. the O/W liquid foundation, which was prepared without adding the dispersant for hydrophobic powder in Comparative Example 10, showed noticeable agglomeration of the hydrophobic powder and inferior feeling on use and water resistance.

[Example 9] O/W cream foundation (cosmetic composition having dispersion blended therein)

An O/W cream foundation was obtained by mixing components in the amounts shown in Table 7 according to the preparation method described below.

[Table 7]

| Composition (wt%) | | Example 9 |
|---|---|---|
| 1 | Crosslinked dimethylpolysiloxane (*1) | 7.0 |
| 2 | Crosslinked dimethylpolysiloxane (*2) | 25.0 |
| 3 | Acrylic silicone resin (*3) | 10.0 |
| 4 | Polyglycerin co-modified branched silicone (*4) | 0.9 |
| 5 | Synthesis Example 1 | 0.4 |
| 6 | POE hydrogenated castor oil | 0.3 |
| 7 | Hydrophobized titanium oxide (*5) | 8.5 |
| 8 | Hydrophobized red iron oxide (*6) | 0.4 |
| 9 | Hydrophobized yellow iron oxide (*6) | 1.0 |
| 10 | Hydrophobized black iron oxide (*6) | 0.1 |
| 11 | 1,3-butylene glycol | 5.0 |
| 12 | Polyacrylamide emulsifier | 1.6 |
| 13 | VP copolymer (5% aqueous solution) | 12.0 |
| 14 | Sodium chloride | 0.1 |
| 15 | Preservative | proper |
| 16 | Fragrance | proper |
| 17 | Purified water | balance |
| Total | | 100.0 |
| Feeling on use | | ◎ |

(continued)

| Composition (wt%) | Example 9 |
|---|---|
| Water resistance | ◎ |
| (*1) Crosslinked dimethylpolysiloxane: KSG-15 (Shin-Etsu Chemical Co., Ltd.)<br>(*2) Crosslinked dimethylpolysiloxane: KSG-16 (Shin-Etsu Chemical Co., Ltd.)<br>(*3) Acrylic silicone resin: KP-545 (Shin-Etsu Chemical Co., Ltd.)<br>(*4) Polyglycerin co-modified branched silicone: KF-6100 (Shin-Etsu Chemical Co., Ltd.)<br>(*5) Hydrophobized titanium oxide: MT-200ST (Tayca Corp.)<br>(*6) Hydrophobized iron oxide powder: treated with 2% KF-9909 (Shin-Etsu Chemical Co., Ltd.) | |

(Preparation method)

Step 1:
mixing a part of component (4), components (5) and (6) with component (11), adding components (7) to (10) thereto, and dispersing the mixture in a part of component (17)
Step 2:
mixing components (1) to (3)
Step 3:
mixing the remainder of (4), components (12) to (15), and the remainder of (17)
Step 4:
adding the mixture of Step 2 to the mixture of Step 3 with stirring, for emulsification, and adding the dispersion of Step 1 and component (16) to the emulsion to yield an O/W cream foundation

The O/W cream foundation of Example 9 was evaluated for feeling on use (fresh and smooth feeling) and water resistance according to the same evaluation method as in Examples 1 to 6. The results are also shown in Table 7. The O/W cream foundation of Example 9 showed a fine texture, satisfactory stability with time, effective spread on the skin, a light finish, non-stickiness, a fresh or light feeling on use, an aesthetically acceptable cosmetic film, water resistance, water repellency, perspiration resistance, long-lasting properties, deterioration resistance, and high stability as demonstrated by no changes with temperature and time.

[Example 10] W/O cream foundation (cosmetic composition having dispersion blended therein)

A W/O cream foundation was obtained by mixing components in the amounts shown in Table 8 according to the preparation method described below.

[Table 8]

| Composition (wt%) | | Example 10 |
|---|---|---|
| 1 | Alkyl-modified crosslinked polyether-modified silicone (*1) | 2.0 |
| 2 | Alkyl-modified crosslinked dimethylpolysiloxane (*2) | 2.0 |
| 3 | Alkyl/polyether-co-modified branched silicone (*3) | 1.0 |
| 4 | Liquid paraffin | 2.0 |
| 5 | Tri(caprylic acid/capric acid) glyceryl | 5.0 |
| 6 | Isotridecyl isononanoate | 9.0 |
| 7 | POE hydrogenated castor oil | 0.3 |
| 8 | Synthesis Example 2 | 0.4 |
| 9 | Hybrid silicone composite powder (*4) | 2.0 |
| 10 | Hydrophobized titanium oxide (*5) | 8.5 |
| 11 | Hydrophobized red iron oxide (*6) | 0.4 |
| 12 | Hydrophobized yellow iron oxide (*6) | 1.0 |

(continued)

| Composition (wt%) | | Example 10 |
|---|---|---|
| 13 | Hydrophobized black iron oxide (*6) | 0.1 |
| 14 | 1,3-butylene glycol | 5.0 |
| 15 | Sodium citrate | 0.2 |
| 16 | Sodium chloride | 0.5 |
| 17 | Preservative | proper |
| 18 | Fragrance | proper |
| 19 | Purified water | balance |
| Total | | 100.0 |
| Feeling on use | | ◎ |
| Water resistance | | ◎ |
| (*1) Alkyl-modified crosslinked polyether-modified silicone: KSG-310 (Shin-Etsu Chemical Co., Ltd.)<br>(*2) Alkyl-modified crosslinked dimethylpolysiloxane: KSG-41 (Shin-Etsu Chemical Co., Ltd.)<br>(*3) Alkyl/polyether-co-modified branched silicone: KF-6038 (Shin-Etsu Chemical Co., Ltd.)<br>(*4) Hybrid silicone composite powder: KSP-100 (Shin-Etsu Chemical Co., Ltd.)<br>(*5) Hydrophobized titanium oxide: STR-100W-OTS (Sakai Chemical Co., Ltd.)<br>(*6) Hydrophobized iron oxide powder: treated with 2% KF-9909 (Shin-Etsu Chemical Co., Ltd.) | | |

(Preparation method)

Step 1:
mixing components (7) and (8) with component (14), adding components (9) to (13) thereto, and dispersing the mixture in a part of component (19)
Step 2:
mixing components (1) to (6)
Step 3:
mixing components (15) to (17) with the remainder of (19)
Step 4:
adding the mixture of Step 3 to the mixture of Step 2 with stirring, for emulsification, and adding the dispersion of Step 1 and component (18) to the emulsion to yield a W/O cream foundation

The W/O cream foundation of Example 10 was evaluated for feeling on use (fresh and smooth feeling) and water resistance according to the same evaluation method as in Examples 1 to 6. The results are also shown in Table 8. The W/O cream foundation of Example 10 showed a fine texture, satisfactory stability with time, effective spread on the skin, a smooth feeling on use, an aesthetically acceptable cosmetic film, water resistance, cold resistance, and long-lasting properties.

[Example 11] W/O stick foundation (cosmetic composition having dispersion blended therein)

A W/O stick foundation was obtained by mixing components in the amounts shown in Table 9 according to the preparation method described below.

[Table 9]

| Composition (wt%) | | Example 11 |
|---|---|---|
| 1 | Ceresin | 5.5 |
| 2 | Inulin stearate (*1) | 2.0 |
| 3 | Neopentyl glycol dicaprate | 8.0 |

(continued)

| Composition (wt%) | | Example 11 |
|---|---|---|
| 4 | Tri(caprylic acid/capric acid) glyceryl | 5.0 |
| 5 | Dimethylpolysiloxane (*2) | 11.5 |
| 6 | Crosslinked polyglycerin-modified silicone (*3) | 4.0 |
| 7 | Alkyl/polyglycerin-co-modified branched silicone (*4) | 1.5 |
| 8 | Spherical polymethylsilsesquioxane powder (*5) | 1.5 |
| 9 | Lecithin | 0.2 |
| 10 | Synthesis Example 1 | 0.3 |
| 11 | Hydrophobized titanium oxide (*6) | 8.5 |
| 12 | Hydrophobized red iron oxide (*7) | 0.4 |
| 13 | Hydrophobized yellow iron oxide (*7) | 1.0 |
| 14 | Hydrophobized black iron oxide (*7) | 0.1 |
| 15 | Dipropylene glycol | 5.0 |
| 16 | Sodium citrate | 0.2 |
| 17 | Sodium chloride | 0.5 |
| 18 | Preservative | proper |
| 19 | Fragrance | proper |
| 20 | Purified water | balance |
| Total | | 100.0 |
| Feeling on use | | ◎ |
| Water resistance | | ◎ |

(*1) Inulin stearate: Leopearl ISK (Chiba Flour Co., Ltd.)
(*2) Dimethylpolysiloxane: KF-96A-6cs (Shin-Etsu Chemical Co., Ltd.)
(*3) Crosslinked polyglycerin-modified silicone: KSG-710 (Shin-Etsu Chemical Co., Ltd.)
(*4) Alkyl/polyglycerin-co-modified branched silicone: KF-6105 (Shin-Etsu Chemical Co., Ltd.)
(*5) Spherical polymethylsilsesquioxane powder: KMP-590 (Shin-Etsu Chemical Co., Ltd.)
(*6) Hydrophobized titanium oxide: ST-455FA (Titan Kogyo, Ltd.)
(*7) Hydrophobized iron oxide powder: treated with 2% KF-9909 (Shin-Etsu Chemical Co., Ltd.)

(Preparation method)

Step 1:
mixing components (9) and (10) with component (15), adding components (11) to (14) thereto, and dispersing the mixture in a part of component (20)
Step 2:
mixing and heating components (1) to (8)
Step 3:
mixing components (16) to (18) with the remainder of (20)
Step 4:
adding the mixture of Step 3 to the mixture of Step 2 with stirring, for emulsification, and adding the dispersion of Step 1 to the emulsion, further adding component (19) thereto, mixing, and casting into a container, obtaining a W/O stick foundation

The W/O stick foundation of Example 11 was evaluated for feeling on use (smooth feeling) and water resistance according to the same evaluation method as in Examples 1 to 6. The results are also shown in Table 9. The W/O stick

foundation of Example 11 showed satisfactory stability with time, effective spread on the skin, a moist smooth feeling on use, an aesthetically acceptable cosmetic film, water resistance, cold resistance, and long-lasting properties.

[Example 12] O/W sunscreen agent (cosmetic composition having dispersion blended therein)

An O/W sunscreen agent was obtained by mixing components in the amounts shown in Table 10 according to the preparation method described below.

[Table 10]

| Composition (wt%) | | Example 12 |
|---|---|---|
| 1 | Alkyl/polyether-co-modified silicone (A-9) (*1) | 1.5 |
| 2 | 1,3-butylene glycol | 14.3 |
| 3 | organically modified clay mineral (*2) | 0.6 |
| 4 | Mineral oil | 14.4 |
| 5 | Acryloyldimethyltaurine ammonium/VP copolymer (5% aqueous solution) (*3) | 7.5 |
| 6 | Synthesis Example 1 | 2.5 |
| 7 | Hydrophobized titanium oxide (*4) | 12.5 |
| 8 | Preservative | proper |
| 9 | Fragrance | proper |
| 10 | Purified water | balance |
| Total | | 100.0 |
| Feeling on use | | ◎ |
| Water resistance | | ◎ |

(*1) Alkyl/polyether-co-modified silicone having the formula (A-9)

[Chem. 46]

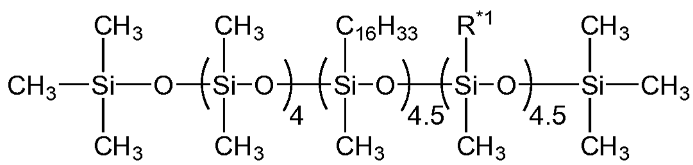

$R^{*1}$: $-C_3H_6O(C_2H_4O)_9H$

[Preparation method]

A reactor was charged with methylhydrogenpolysiloxane (H-7) and alkene (ii-2), after which a platinum catalyst was added, and reaction was carried out at 80°C. The reactor was then charged with polyoxyalkylene monoallyl ether (iv-1), after which isopropyl alcohol and a platinum catalyst were added, and reaction was carried out at 80°C. The isopropyl alcohol was removed by heating under reduced pressure, obtaining alkyl/polyether-co-modified silicone. The amount of $R^2$ was 25% by weight of the total weight of the organopolysiloxane. The hydrophilic-lipophilic balance (HLB) defined as (total molecular weight of hydrophilic moiety)/(total molecular weight)x20 was 10.

(H-7)

[Chem. 47]

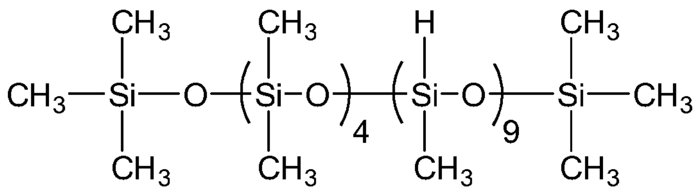

(ii-2)

[Chem. 48] $CH_2=CH-C_{14}H_{29}$

(iv-1)

[Chem. 49]

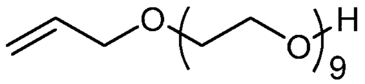

(Preparation method)

Step 1:
mixing component (1), a part of component (2), component (3), and a part of component (4)
Step 2:
mixing a part of component (2) with components (6) and (7), and dispersing the mixture in a part of component (10)
Step 3:
adding the remainder of component (2) and the remainder of component (4) to the mixture of Step 1 with stirring, and adding components (5) and (8) and the remainder of component (10) thereto, to form a mixture
Step 4:
adding the dispersion of step 2 to the mixture of step 3 with stirring, further adding component (9) thereto, and mixing them to form an O/W sunscreen agent

The O/W sunscreen agent of Example 12 was evaluated for feeling on use (smooth feeling) and water resistance according to the same evaluation method as in Examples 1 to 6. The results are also shown in Table 10. The O/W sunscreen agent of Example 12 showed satisfactory stability with time, effective spread on the skin, a moist smooth feeling on use, an aesthetically acceptable cosmetic film, water resistance, cold resistance, and long-lasting properties.

[Example 13] O/W sunscreen agent (cosmetic composition having dispersion blended therein)

An O/W sunscreen agent was obtained by mixing the components in the amounts shown in Table 11 according to the preparation method described below.

[Table 11]

| Composition (wt%) | | Example 13 |
|---|---|---|
| 1 | Alkyl/polyether-co-modified silicone (A-9) (*1) | 2.0 |

(continued)

| Composition (wt%) | | Example 13 |
|---|---|---|
| 2 | 1,3-butylene glycol | 16.0 |
| 3 | Hydrogenated polyisobutene | 10.7 |
| 4 | Isotridecyl isononanoate | 5.0 |
| 5 | Dimethicone | 2.0 |
| 6 | Isostearic acid | 0.3 |
| 7 | Acryloyldimethyltaurine ammonium/VP copolymer (5% aqueous solution) (*2) | 0.6 |
| 8 | NaOH (1% aqueous solution) | 4.2 |
| 9 | Synthesis Example 1 | 3.4 |
| 10 | Hydrophobized titanium oxide (*3) | 10.0 |
| 11 | Hydrophobized zinc oxide (*4) | 14.0 |
| 12 | Preservative | proper |
| 13 | Fragrance | proper |
| 14 | Purified water | balance |
| Total | | 100.0 |
| Feeling on use | | ◎ |
| (*1) Alkyl/polyether-co-modified silicone (A-9) as used above<br>(*2) Acryloyldimethyltaurine ammonium/VP copolymer: Aristoflex AVC (Clariant AG)<br>(*3) Hydrophobized titanium oxide: STR-100W-OTS (Sakai Chemical Co., Ltd.)<br>(*4) Hydrophobized zinc oxide: FINEX-30-OTS (Sakai Chemical Co., Ltd.) | | |

(Preparation method)

Step 1:
mixing a part of component (2) with components (9) to (11), and dispersing the mixture in a part of component (14)
Step 2:
mixing component (7) and a part of component (14)
Step 3:
mixing component (1) and the remainder of component (2) with stirring, adding components (3) to (6) to the mixture, and further adding component (8) and the remainder of component (14) thereto to form a mixture
Step 4:
adding the mixture of step 2 and the mixture of step 1 to the mixture of step 3 with stirring, further adding components (12) and (13) thereto, and mixing them to form an O/W sunscreen agent

The O/W sunscreen agent of Example 13 was evaluated for feeling on use (smooth feeling) and water resistance according to the same evaluation method as in Examples 1 to 6. The results are also shown in Table 11. The O/W sunscreen agent of Example 13 showed satisfactory stability with time, effective spread on the skin, a moist smooth feeling on use, an aesthetically acceptable cosmetic film, water resistance, cold resistance, and long-lasting properties.

## Claims

**1.** An organopolysiloxane containing (poly)glycerin and polyoxyalkylene groups, represented by the compositional formula (1): [Chem. 1]

$$(R^1_3SiO_{1/2})_{2+f+2g}(R^1_2SiO_{2/2})_a(R^1R^2SiO_{2/2})_b(R^1R^3SiO_{2/2})_c(R^1R^4SiO_{2/2})_d(R^1R^5SiO_{2/2})_e(R^1SiO_{3/2})_f(SiO_{4/2})_g \quad (1)$$

wherein $R^1$ is independently a monovalent hydrocarbon group selected from $C_1$-$C_5$ alkyl groups, $C_6$-$C_{15}$ aryl groups, and $C_7$-$C_{15}$ aralkyl groups,

$R^2$ is a $C_6$-$C_{20}$ alkyl group,
$R^3$ is a (poly)glycerin group having the general formula (2):

[Chem. 2]

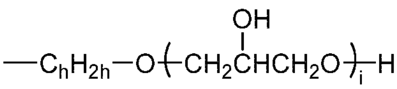 (2)

wherein h is an integer satisfying $0 \leq h \leq 20$, and i satisfies $1 \leq i \leq 10$, $R^4$ is a polyoxyalkylene group having the general formula (3):
[Chem. 3]

$$-C_jH_{2j}-O-(C_2H_4O)_{k1}-(C_3H_6O)_{k2}-R^6 \qquad (3)$$

wherein $R^6$ is hydrogen or a $C_1$-$C_{30}$ hydrocarbon group, j is an integer satisfying $0 \leq j \leq 20$, k1 and k2 satisfy $2 \leq k1 \leq 100$ and $0 \leq k2 \leq 100$,
$R^5$ is a group having the general formula (4), (5), (6) or (7):
[Chem. 4]

$$-(CH_2)_2-C_mH_{2m}-(SiOR^1{}_2)_n-SiR^1{}_3 \qquad (4)$$

$$-(CH_2)_2-C_mH_{2m}-SiR^1{}_{n1}-(OSiR^1{}_3)_{3-n1} \qquad (5)$$

$$-(CH_2)_2-C_mH_{2m}-SiR^1{}_{n1}-(OSiR^1{}_{n2}(OSiR^1{}_3)_{3-n2})_{3-n1} \qquad (6)$$

$$-(CH_2)_2-C_mH_{2m}-SiR^1{}_{n1}-(OSiR^1{}_{n1}-(OSiR^1{}_{n2}(OSiR^1{}_{n3})_{3-n3})_{3-n2})_{3-n1} \qquad (7)$$

wherein $R^1$ is as defined above, m and n each are an integer satisfying $0 \leq m \leq 5$ and $0 \leq n \leq 100$, n1 to n3 each are an integer satisfying $0 \leq n1 \leq 2$, $0 \leq n2 \leq 2$, and $0 \leq n3 \leq 2$, a, b, c, d, e, f, and g each satisfy

$$0 \leq a \leq 50,$$

$$0 \leq b \leq 20,$$

$$1 \leq c \leq 10,$$

$$1 \leq d \leq 10,$$

$$0 \leq e \leq 10,$$

$$f \geq 0, \text{ and } g \geq 0.$$

**2.** The organopolysiloxane of claim 1 wherein in compositional formula (1), the ratio of c/d is $0.1 \leq (c/d) \leq 1.5$.

**3.** A treated hydrophobic powder comprising (B) a hydrophobic powder which is treated with (A) the organopolysiloxane of claim 1 or 2.

**4.** A dispersion comprising the treated hydrophobic powder of claim 3.

**5.** A cosmetic composition comprising the treated hydrophobic powder of claim 3 or the dispersion of claim 4.

**6.** The cosmetic composition of claim 5, further comprising (D) a polyhydric alcohol.

**7.** The cosmetic composition of claim 5, further comprising (E) an oil.

**INTERNATIONAL SEARCH REPORT**

International application No. **PCT/JP2022/047303**

**A. CLASSIFICATION OF SUBJECT MATTER**

***C08G 77/46***(2006.01)i; ***A61K 8/04***(2006.01)i; ***A61Q 1/00***(2006.01)i; ***A61Q 5/00***(2006.01)i; ***A61Q 17/04***(2006.01)i; ***A61Q 19/00***(2006.01)i; ***C08G 77/38***(2006.01)i

FI: C08G77/46; A61K8/04; A61Q1/00; A61Q5/00; A61Q17/04; A61Q19/00; C08G77/38

According to International Patent Classification (IPC) or to both national classification and IPC

**B. FIELDS SEARCHED**

Minimum documentation searched (classification system followed by classification symbols)

C08G77/46; A61K8/04; A61Q1/00; A61Q5/00; A61Q17/04; A61Q19/00; C08G77/38

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| A | WO 2019/107497 A1 (SHIN-ETSU CHEMICAL CO., LTD.) 06 June 2019 (2019-06-06) entire text | 1-7 |
| A | JP 2008-169176 A (SHIN-ETSU CHEMICAL CO., LTD.) 24 July 2008 (2008-07-24) entire text | 1-7 |
| A | JP 2010-30954 A (FUJI KASEI KK) 12 February 2010 (2010-02-12) entire text | 1-7 |
| A | JP 2021-526180 A (SHIN-ETSU CHEMICAL CO., LTD.) 30 September 2021 (2021-09-30) entire text | 1-7 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **26 January 2023** | **14 February 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2022/047303**

| Patent document cited in search report | Publication date (day/month/year) | Patent family member(s) | Publication date (day/month/year) |
|---|---|---|---|
| WO 2019/107497 A1 | 06 June 2019 | US 2020/0332065 A1<br>entire text<br>EP 3719056 A1<br>CN 111433256 A<br>KR 10-2020-0093599 A | |
| JP 2008-169176 A | 24 July 2008 | US 2008/0171686 A1<br>entire text<br>EP 1944330 A1 | |
| JP 2010-30954 A | 12 February 2010 | (Family: none) | |
| JP 2021-526180 A | 30 September 2021 | US 2019/0365632 A1<br>entire text<br>WO 2019/235294 A1<br>CN 112236465 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2018024881 A
- WO 2016056589 A
- JP 2017137252 A
- JP 2018115211 A